(19) **Europäisches Patentamt**
**European Patent Office**
**Office européen des brevets**

(11) **EP 3 998 480 B1**

(12) **EUROPEAN PATENT SPECIFICATION**

(45) Date of publication and mention
of the grant of the patent:
**20.05.2026 Bulletin 2026/21**

(21) Application number: **20836301.0**

(22) Date of filing: **09.07.2020**

(51) International Patent Classification (IPC):
**G01N 33/68** (2006.01)   **G01N 30/72** (2006.01)
**G16B 40/10** (2019.01)   **G06N 3/02** (2006.01)
**G01N 30/88** (2006.01)

(52) Cooperative Patent Classification (CPC):
**G01N 33/57525; G06N 3/0499; G06N 3/09;**
**G06N 3/0985;** G01N 2800/60; G06N 3/048;
G06N 3/082; G06N 20/10; G16B 40/20

(86) International application number:
**PCT/KR2020/008979**

(87) International publication number:
**WO 2021/006649 (14.01.2021 Gazette 2021/02)**

(54) **BIOMARKER PANEL FOR DIAGNOSING PANCREATIC CANCER AND USE THEREOF**

BIOMARKERPANEL ZUR DIAGNOSE VON BAUCHSPEICHELDRÜSENKREBS UND SEINE
VERWENDUNG

PANEL DE BIOMARQUEURS POUR LE DIAGNOSTIC DU CANCER DU PANCRÉAS ET
UTILISATION DE CELUI-CI

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **09.07.2019 KR 20190082497**

(43) Date of publication of application:
**18.05.2022 Bulletin 2022/20**

(73) Proprietor: **Bertis Inc**
**Seongnam-si, Gyeonggi-do 13605 (KR)**

(72) Inventors:
• **KIM, Youngsoo**
**Seoul 06284 (KR)**
• **KIM, Yoseop**
**Incheon 22003 (KR)**
• **SON, Minsoo**
**Seoul 02861 (KR)**
• **JANG, Jin-Young**
**Seoul 03080 (KR)**

(74) Representative: **Schnappauf, Georg**
**ZSP Patentanwälte PartG mbB**
**Hansastraße 32**
**80686 München (DE)**

(56) References cited:
**WO-A1-2015/157557**     **WO-A1-2016/195051**
**WO-A1-2017/180735**     **WO-A1-2021/076036**
**KR-A- 20140 002 149**     **KR-A- 20160 045 547**
**US-A1- 2015 018 235**

• **HERREROS-VILLANUEVA MARTA ET AL: "Non-**
**invasive biomarkers in pancreatic cancer**
**diagnosis: what we need versus what we have",**
ANNALS OF TRANSLATIONAL MEDICINE, vol. 4,
no. 7, 1 April 2016 (2016-04-01), US, pages 134 -
134, XP093050274, ISSN: 2305-5839, DOI:
10.21037/atm.2016.03.44
• **TOSHIHIRO YONEYAMA ET AL: "Identification of**
**IGFBP2 and IGFBP3 As Compensatory**
**Biomarkers for CA19-9 in Early-Stage Pancreatic**
**Cancer Using a Combination of Antibody-Based**
**and LC-MS/MS-Based Proteomics", PLOS ONE,**
**vol. 11, no. 8, 31 August 2016 (2016-08-31), pages**
**1 - 23, XP055623544, DOI: 10.1371/**
**journal.pone.0161009**

EP 3 998 480 B1

**(Cont. next page)**

- **CHEN JIONG ET AL: "Identification and verification of transthyretin as a potential biomarker for pancreatic ductal adenocarcinoma", vol. 139, no. 7, 1 July 2013 (2013-07-01), pages 1117 - 1127, XP009502172, ISSN: 0171-5216, Retrieved from the Internet <URL:http://link.springer.com/10.1007/s00432-013-1422-4> [retrieved on 20130402], DOI: 10.1007/S00432-013-1422-4**

- **JINA KO, BALDASSANO STEVEN N., LOH PO-LING, KORDING KONRAD, LITT BRIAN, ISSADORE DAVID: "Machine learning to detect signatures of disease in liquid biopsies – a user's guide", LAB ON A CHIP, ROYAL SOCIETY OF CHEMISTRY, vol. 18, no. 3, 1 January 2018 (2018-01-01), pages 1 - 21, XP055703589, ISSN: 1473-0197, DOI: 10.1039/C7LC00955K**

**Description**

[Technical Field]

The present invention relates to a method for diagnosing pancreatic cancer, comprising: measuring the expression level of a combination of biomarkers from blood isolated from a subject; and correlating with pancreatic cancer by comparing the measurement result with a measurement result corresponding to the corresponding marker of a control sample, wherein the combination of biomarkers is TTHY, C4BPB, CLU, C1R, ECM1, SERPINA5 (IPSP), LDHB, COL4A2, SERPINC1, VIM, ICAM1, IGFBP2, C4BPA, C5, PPBP, C1S, LRG1, APOH, C7 and IGFBP3; and wherein the correlating with pancreatic cancer further comprises determining the subject to have pancreatic cancer when the expression level increases or decreases in comparison with the expression level measured in the control, as a result of measuring the subject, the increasing marker is C1R, CIS, C4BPA, C4BPB, C5, C7, ICAM1, IGFBP2, LDHB, LRG1, PPBP, SERPINC1 and VIM, and the decreasing marker is APOH, CLU, COL4A2, ECM1, IGFBP3, IPSP, and TTHY; and wherein the measuring the expression level is detecting the expression level of the biomarker at a protein or nucleic acid level, wherein the protein level detection is carried out by western blot, ELISA, radioimmunoassay, immunodiffusion, immunoelectrophoresis, tissue immunostaining, immunoprecipitation assay, complement fixation assay, FACS, mass spectrometry, or protein array; and wherein the nucleic acid detection is carried out by polymerase chain reaction, reverse transcription-polymerase chain reaction, competitive polymerase chain reaction, nuclease protection analysis (RNase, S 1 nuclease assay), in situ cross method, nucleic acid microarray or northern blot.

[Background Art]

According to 2015 national cancer registration statistics, pancreatic cancer is an important disease that accounts for the 8th highest incidence of cancer in Korea, and the incidence thereof is steadily increasing. Currently, surgery is the only curative treatment method for pancreatic cancer because no effective anticancer drug has been developed for pancreatic cancer to date. However, since pancreatic cancer has no characteristic symptoms and there is no early diagnosis method, about 20% of patients with pancreatic cancer can actually undergo surgery, and the remaining 80% or more of the patients are diagnosed with untreatable terminal cancer. Further, surgical resection is the best treatment method in stages I and II, which correspond to early-stage pancreatic cancer, but the survival rate of patients with pancreatic cancer, who have undergone surgical resection, appears to be about 10 to 20%. Pancreatic cancer is a cancer that has the worst prognosis, it is difficult to detect pancreatic cancer because the pancreas is a retroperitoneal organ, and in spite of a small cancer with a size of 2 cm or less, the motility of pancreatic cancer cells is so high that pancreatic cancer immediately infiltrates the surrounding blood vessels, gastrointestinal tract, nerves, and the like, and metastasizes to nearby lymph nodes, the liver, or the like. Therefore, it is very important to make a regular and early diagnosis of pancreatic cancer and predict the severity of disease.

The diagnosis of pancreatic cancer includes an examination using tumor markers (CA19-9), an imaging examination (abdominal ultrasonography, abdominal CT, magnetic resonance imaging MRI, endoscopic ultrasonography, endoscopic retrograde cholangiopancreatography, PET, and diagnostic laparoscopy), tissue biopsy, and the like. However, the aforementioned diagnostic methods have reduced accuracy and are very inconvenient because patients experience pain caused by the procedures. Therefore, it is required to develop a test method capable of easily and quickly diagnosing pancreatic cancer, and there is a need for a new blood test-based diagnostic marker which is non-invasive, rapid, and improves sensitivity/specificity. Accordingly, biomarkers capable of increasing the prognosis of pancreatic cancer have been activity studied.

Currently, CA19-9 is present as a marker for diagnosing pancreatic cancer, which has been approved by the US FDA. CA19-9 is known to have a sensitivity of about 79% and a specificity of 82% as a whole. However, 10 to 15% of patients with pancreatic cancer do not express CA19-9 due to the lack of Lewis A antigen, and it is known that CA19-9 can also increase in liver cancer, gastric cancer, lung cancer, ovarian cancer, and thyroid cancer in addition to pancreatic cancer. Therefore, there are limitations on the diagnosis of pancreatic cancer with CA19-9 alone. In addition, although the diagnostic standard for performing a selective test for pancreatic cancer using CA19-9 is specified as 37 U/mL, according to research results, it was confirmed that when asymptomatic patients are tested using 37 U/mL as a standard value, the positive predictive value of CA 19-9 for malignant tumors is 0.5% for pancreatic cancer and 3.4% for other cancerous diseases, which are very low. That is, the CA19-9 test is extremely ineffective for diagnosing pancreatic cancer in asymptomatic patients Chen Jiong et al.;"Identification and verification of transthyretin as a potential biomarker for pancreatic ductal adenocarcinoma",JOURNAL OF CANCER RESEARCH AND CLINICAL ONCOLOGY, SPRINGER INTERNATIONAL, BERLIN, DE,vol. 139, no. 7 1 July 2013 (2013-07-01), pages 1117-1127, discloses methods to diagnose pancreatic cancer.

Korean Patent Publication No. 2015-0030046 discloses a method for providing information for diagnosing pancreatic cancer by analyzing the level of the complement factor I protein in an individual sample as a biomarker for diagnosing pancreatic cancer and a composition for diagnosing pancreatic cancer, including a polypeptide or antibody which specifically binds to the complement factor I protein.

Therefore, there is a need for developing a marker for diagnosing pancreatic cancer capable of conveniently and accurately diagnosing the onset, possibility or risk of pancreatic cancer at an early stage.

[Disclosure]

[Technical Problem]

The present application has been made in an effort to provide a method for diagnosing pancreatic cancer. Hereinafter, the termmology "embodiment" relates to the disclosure and only forms part of the invention if it falls within the scope of the claims.

[Technical Solution]

In one aspect, the present application provides a biomarker panel or composition for diagnosing pancreatic cancer, including a material for measuring the expression level or expression level of a combination of two or more markers selected from Table 1.

In an exemplary embodiment, the combination of two or more markers are described in the claims, Tables 5, 8, and 9-1 and 9-2 of the present application.

In another exemplary embodiment, each combination of the biomarkers according to the present application may further include an existing pancreatic cancer marker CA19-9.

In still another exemplary embodiment, a reagent capable of detecting the expression level of each biomarker according to the present application at a protein or nucleic acid level is included.

In an exemplary embodiment, protein expression levels are particularly performed by mass spectrometry, which includes tandem mass spectrometry, ion trap mass spectrometry, triple quadrupole mass spectrometry, hybrid ion trap/quadrupole mass spectrometry or time-of-flight mass spectrometry.

In an exemplary embodiment, a mode used for the mass spectrometry is selected reaction monitoring (SRM) or multiple reaction monitoring (MRM), particularly MRM.

In an exemplary embodiment, peptides used for MRM analysis are shown in Table 2, and in the case of some proteins, one or more peptides may be used.

In another aspect, the present application provides a method for detecting a biomarker for diagnosing pancreatic cancer *in vitro,* the method including: measuring the expression level of one or more biomarker panels according to Tables 5, 8, and 9-1 and 9-2 from blood isolated from a subject; and correlating with pancreatic cancer by comparing the measurement result with a measurement result corresponding to the corresponding marker of a control sample, in order to provide information necessary for the diagnosis of pancreatic cancer.

In an exemplary embodiment of the method according to the present application, the combination of each biomarker may further include CA19-9.

In an exemplary embodiment of the method according to the present application, the correlating with pancreatic cancer further includes determining the subject to have pancreatic cancer when the expression level increases or decreases in comparison with the expression level measured in the control, as a result of measuring the subject, the increasing marker is ADIPO, AGT, BTD, C1R, C1S, C4BPA, C4BPB, C5, C6, CDH11, CFH, CFI, C7, CPN2, CTSD, FCGBP, FSTL1, GSTP1, HSPG2, ICAM1, IFRD1, IGFBP2, ITIH4, LDHB, LRG1, MBL2, MMSA, P4HB, PKM2, PPBP, PROS1, SERPINC1, SOD3, SPARC, THBS1, TXN, and VIM, and the decreasing marker is APOC1, APOH, CAP1, CLU, COL4A2, CORO1C, ECM1, HRG, IGFBP3, SERPINA5, ITIH2, KLKB1, PDCD4, PTPRJ, SEPP1, SFTPB, and TTHY.

In an exemplary embodiment of the method according to the present application, in the measuring of the expression level, the expression level of each biomarker is measured by mass spectrometry including tandem mass spectrometry, ion trap mass spectrometry, triple quadrupole mass spectrometry, hybrid ion trap/quadrupole mass spectrometry or time-of-flight mass spectrometry.

In an exemplary embodiment of the method according to the present application, a mode used for the mass spectrometry is selected reaction monitoring (SRM) or multiple reaction monitoring (MRM), particularly MRM.

In an exemplary embodiment of the method according to the present application, peptides used for the MRM analysis are shown in Table 2, and in the case of some proteins, one or more peptides may be used.

In another aspect, the present application relates to a method for selecting/constructing a combination of markers necessary for constructing a biopanel capable of exhibiting an optimum effect for diagnosing pancreatic cancer from various markers as shown in Table 1.

In an exemplary embodiment, the method is a method for constructing a biomarker panel for diagnosing pancreatic cancer, the method including: obtaining expression level data of a plurality of biomarkers using mass spectrometry in blood isolated from a subject, wherein the plurality of biomarkers has a change in expression level in patients with pancreatic cancer compared to an expression level of a control, and the control is normal and benign pancreatic tumors; and selecting a combination of biomarkers for constructing a biomarker panel for diagnosing pancreatic cancer based on data of a plurality of expression levels obtained from the above step using deep learning, logistic regression or a support vector machine (SVM).

In an exemplary embodiment, the deep learning is a Feed-Forward neural network of an H2o package and a hyperparameter used for the deep learning satisfies the following criteria: Activation function formula (1): ReLU (z) = max (0, z); the number of hidden layers and the number of neurons of the hidden layers are 2 and 20, respectively; a learning method is an ADADELTA optimizer, or the number of learning repetitions (epochs) is 400, L2 normalization and a dropout for preventing an overfitting are 1E-5 and 0.5, respectively; the number of samples at one learning is -2; and a Max w2 is 10.

In another exemplary embodiment, a hyperparameter of the SVM satisfies the following criteria: Kernel: Gaussian radial basis function (RBF); Gamma: 0.15; and cost: 2.

In still another exemplary embodiment, the method further includes: a data preprocessing step, in which when the deep learning is used, expression level data is excluded after the obtaining of the expression level data of the plurality of biomarkers determined by using the mass spectrometry, the expression level data satisfying standards such as a cutoff value of more than 10% of a coefficient of variation (CV) for three repetitive analysis results for one sample; and/or a ratio of a peptide to be tested/an internal standard (SIS) peptide of 0.1 to 10; and/or a peptide affected by a confounding factor, which is a bias according to a sample batch to be analyzed.

In an exemplary embodiment, the expression level is measured by mass spectrometry including tandem mass spectrometry, ion trap mass spectrometry, triple quadrupole mass spectrometry, hybrid ion trap/quadrupole mass spectrometry or time-of-flight mass spectrometry. In an exemplary embodiment of the method according to the present application, a mode used for the mass spectrometry is selected reaction monitoring (SRM) or multiple reaction monitoring (MRM).

In an exemplary embodiment, the mass spectrometry is in the MRM mode, and the plurality of biomarkers and peptides of each biomarker used in the mass spectrometry are shown as in Table 2.

[Advantageous Effects]

The markers according to the present application and methods using the same can be used to predict the onset and diagnosis of pancreatic cancer with further improved performance. Further, when mass analysis-based multiple reaction monitoring (MRM) is used, the MRM can be easily put into practical use as a method of testing peptides with MRM instead of the existing immunoassay method in the clinical field of hospitals, and can be utilized as a method of diagnosing pancreatic cancer with more improved accuracy in combination with a CA19-9 test which is an immunoassay kit method. The marker according to the present application is a non-invasive diagnostic method using blood and is very useful for early detection at home and general clinics. Since the pain and financial burden on a target patient can be reduced and furthermore, pancreatic cancer can be detected by a simple blood test at the time of a health examination, it is possible to bring about a medical cost reduction effect from a national point of view.

[Description of Drawings]

FIG. 1 is a schematic view of a perceptron according to one exemplary embodiment of the present application.

FIG. 2 shows the function formula and graph of an activation function ReLU according to one exemplary embodiment of the present application.

FIG. 3 is a schematic view of an MRM technology according to one exemplary embodiment of the present application.

FIG. 4 is a graph showing examples of MRM relative quantitative peaks according to one exemplary embodiment of the present application.

FIG. 5 is a set of graphs showing the calibration curve results according to one exemplary embodiment of the present application.

FIG. 6 is a flowchart showing a process of selecting a reproducible marker candidate group according to one embodiment of the present application.

FIG. 7 is a schematic view showing a configuration example of a deep learning network according to one exemplary embodiment of the present application.

FIG. 8 is a flowchart showing a deep learning network optimization process according to one exemplary embodiment of the present application.

FIG. 9 is a set of graphs showing the main hyperparameter optimization results of the deep learning network according to one exemplary embodiment of the present application.

FIG. 10 is a distribution graph of skewness before and after data transformation according to one exemplary embodiment of the present application.

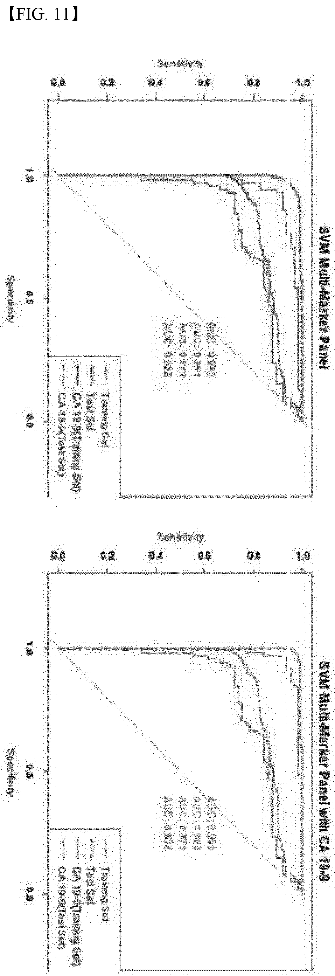

FIG. 11 shows the ROC analysis results of the multi-marker panel in the training set and test set according to one exemplary embodiment of the present application.

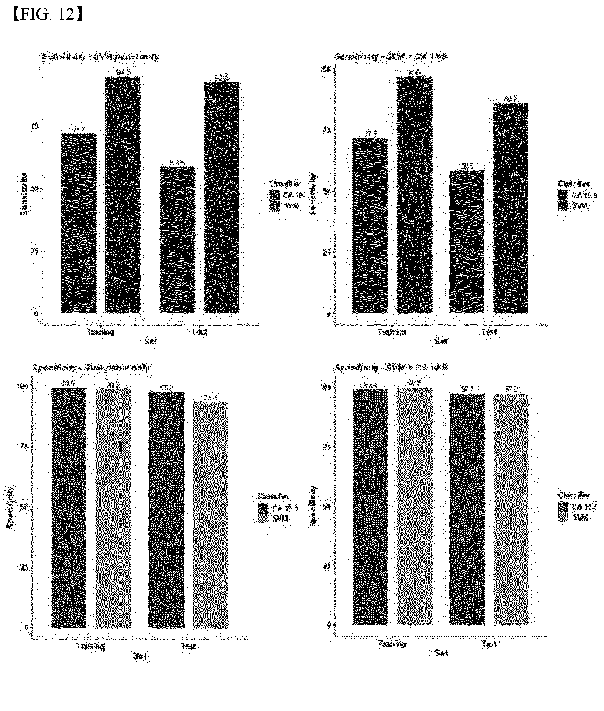

FIG. 12 shows the sensitivity and specificity results of the multi-marker panel in the training set and test set according to one exemplary embodiment of the present application.

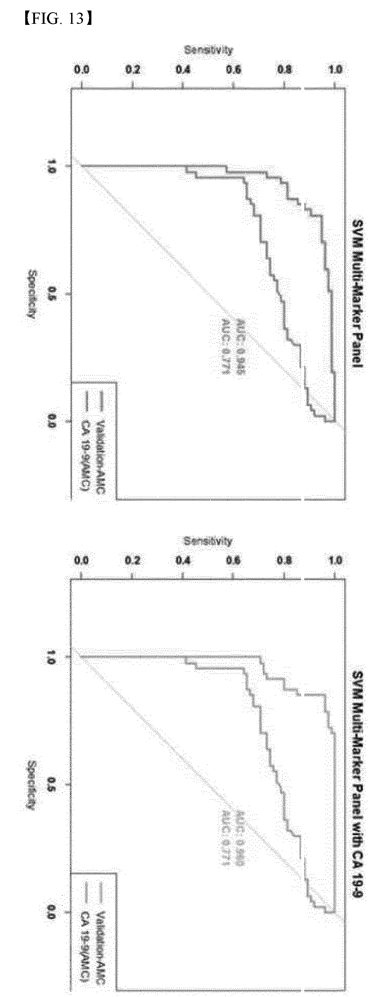

FIG. 13 shows the ROC analysis results of the multi-marker panel in an independent validation set according to one exemplary embodiment of the present application.

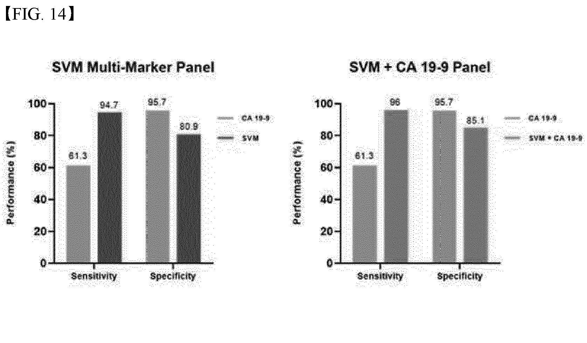

FIG. 14 shows the sensitivity and specificity results of the multi-marker panel in an independent validation set according to one exemplary embodiment of the present application.

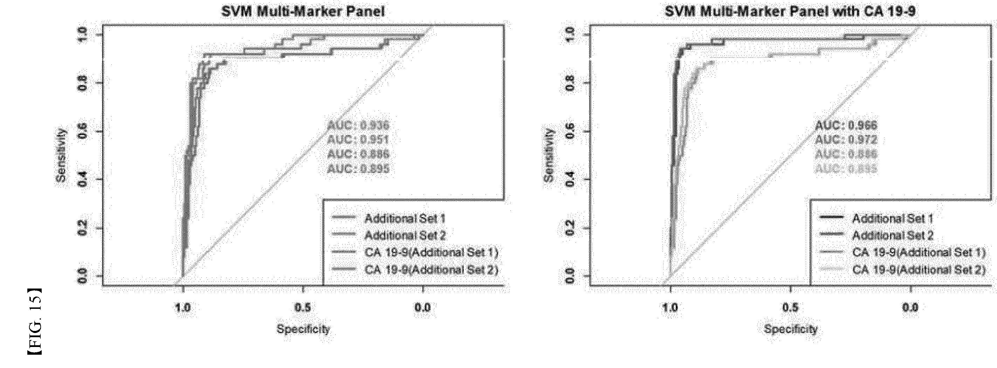

FIG. 15 shows the ROC analysis results of the multi-marker panel in Additional Cohorts 1 and 2 according to one exemplary embodiment of the present application.

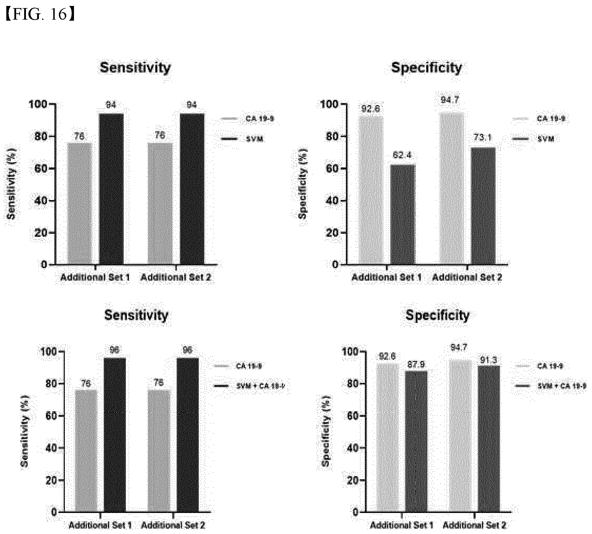

FIG. 16 shows the sensitivity and specificity results of the multi-marker panel in Additional Cohorts 1 and 2 according to one exemplary embodiment of the present application.

[Modes of the Invention]

The present application is based on the development of a biomarker panel capable of diagnosing pancreatic cancer, which shows a differential expression compared to a control in a biological sample derived from patients with pancreatic cancer.

In one aspect, the present application relates to a biomarker or a combination of biomarkers for diagnosing pancreatic cancer shown in the following Table 1.

[Table 1]

| Gene Symbol | Uniprot protein name | Gene Symbol | Uniprot protein name |
|---|---|---|---|
| ADIPOQ | Adiponectin | HSPG2 | Basement membrane-specific heparan sulfate proteoglycan core protein |
| AGT | Angiotensinogen | ICAM1 | Intercellular adhesion molecule 1 |
| ALDH6A1 (MMSA) | Methylmalonate-semialdehyde dehydrogenase | IFRD1 | Interferon-related developmental regulator 1 |
| APOC1 | Apolipoprotein C-I | IGFBP2 | Insulin-like growth factor-binding protein 2 |
| APOH | Beta-2-glycoprotein 1 | IGFBP3 | Insulin-like growth factor-binding protein 3 |
| BTD | Biotinidase | ITIH2 | Inter-alpha-trypsin inhibitor heavy chain H2 |
| C1R | Complement C1r subcomponent | ITIH4 | Inter-alpha-trypsin inhibitor heavy chain H4 |
| C1S | Complement C1s subcomponent | KLKB1 | Plasma kallikrein |
| C4BPA | C4b-binding protein alpha chain | LDHB | L-lactate dehydrogenase B chain |
| C4BPB | C4b-binding protein beta chain | LRG1 | Leucine-rich alpha-2-glycoprotein |
| C5 | Complement C5 | MBL2 | Mannose-binding protein C |
| C6 | Complement component C6 | P4HB | Protein disulfide-isomerase |
| C7 | Complement component C7 | PDCD4 | Programmed cell death protein 4 |
| CAP1 | Adenylyl cyclase-associated protein 1 | PKM2 | Pyruvate kinase |
| CDH11 | Cadherin-11 | PPBP | Platelet basic protein |
| CFH | Complement factor H | PROS1 | Vitamin K-dependent protein S |
| CFI | Complement factor I | PTPRJ | Receptor-type tyrosine-protein phosphatase eta |
| CLU | Clusterin | SEPP1 | Selenoprotein P |
| COL4A2 | Collagen alpha-2(IV) chain | SERPINA5 (IPSP) | Plasma serine protease inhibitor |
| CORO1C | Coronin-1C | SERPINC1 | Antithrombin-III |
| CPN2 | Carboxypeptidase N subunit 2 | SFTPB | Pulmonary surfactant-associated protein B |
| CTSD | Cathepsin D | SOD3 | Extracellular superoxide dismutase |
| ECM1 | Extracellular matrix protein 1 | SPARC | SPARC |
| FCGBP | IgGFc-binding protein | THBS1 | Thrombospondin-1 |
| FSTL1 | Follistatin-related protein 1 | TTHY | Transthyretin |
| GSTP1 | Glutathione S-transferase P | TXN | Thioredoxin |
| HRG | Histidine-rich glycoprotein | VIM | Vimentin |

In the present application, pancreatic cancer includes pancreatic ductal adenocarcinoma, acinar cell carcinoma, and neuroendocrine, which are malignant tumors, and serous cystadenoma, a mucinous cystic neoplasm, an intraductal papillary mucinous neoplasm (IPMN), and a solid pseudopapillary tumor, which are cystic benign tumors, and also includes pancreatic cancer according to the progression stages of cancer classified as Stage I, Stage II, and the like.

The marker according to the present application may be an indicator for the onset and progression of pancreatic cancer, and may be used for the onset of pancreatic cancer, the progression of the disease, and the diagnosis or prognosis of the disease. In addition, the biomarker according to the present application may diagnose pancreatic cancer as early as possible at a stage where curative resection of pancreatic cancer is possible. Furthermore, the marker protein of the present application may be used for a method for providing information necessary of the diagnosis of pancreatic cancer using a composition or kit for diagnosing pancreatic cancer.

In the present application, diagnosis includes determining the susceptibility of a subject for a specific disease or disorder, that is, a subject to be tested, determining whether a subject currently has a specific disease or disorder, determining the prognosis of a subject with a specific disease or disorder (for example, identification of a pre-metastatic or metastatic cancer state, determining the stage of the cancer, or determining the responsiveness of the cancer to treatment), or therametrics (for example, monitoring the condition of a subject in order to provide information on therapeutic efficacy).

In the present invention, the term "diagnostic marker or diagnosis marker" refers to a material capable of diagnosing pancreatic cancer by distinguishing the presence of pancreatic cancer cells from normal cells, and includes a protein or nucleic acid which shows an increase (ADIPO, AGT, BTD, C1R, C1S, C4BPA, C4BPB, C5, C6, CDH11, CFH, CFI, C7, CPN2, CTSD, FCGBP, FSTL1, GSTP1, HSPG2, ICAM1, IFRD1, IGFBP2, ITIH4, LDHB, LRG1, MBL2, MMSA, P4HB, PKM2, PPBP, PROS1, SERPINC1, SOD3, SPARC, THBS1, TXN, VIM) or decrease (APOC1, APOH, CAP1, CLU, COL4A2, CORO1C, ECM1, HRG, IGFBP3, SERPINA5, ITIH2, KLKB1, PDCD4, PTPRJ, SEPP1, SFTPB, TTHY) pattern in a tissue or site having pancreatic cancer compared to normal cells. The amino acid and gene sequences of each protein described in Table 1 can be searched for in Uniprot.

In an exemplary embodiment, particularly, the markers may be used as a biomarker panel including two or more combinations to be utilized as a method of improving a discriminative power (specificity and/or sensitivity) capable of distinguishing the diagnosis and/or prognosis of patients with pancreatic cancer from a normal control and the progression of pancreatic cancer.

As used herein, the term "biomarker panel" is constructed using any combination of biomarkers for the diagnosis of pancreatic cancer. These combinations may mean an entire set, or any subset or subcombination thereof. The biomarker panel according to the present application may include 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53 or 54 biomarkers. In the present application, the biomarker panel may be a biomarker panel or biomarker panel composition including a material for detecting each marker included in the panel.

In an exemplary embodiment, the combination of markers according to the present application is one or more selected from the group consisting of: TTHY, ITIH4, CLU, SEPP1, LRG1, KLKB1, C1R, SERPINA5, BTD, IGFBP2, C5, PROS1 and CFI; LRG1, PPAP, C5, KLKB1, CLU, IGFBP2, THBS1, IFRD1, SERPINA5, TTHY, ICAM1, and VIM; CLU, C5, KLKB1, PPBP, IFRD1, IGFBP2, ICAM1, C4BPA, PTPRJ, ECM1, VIM, C4BPB, SERPINA5 and TTHY, CLU, C5, KLKB1, PPBP, ICAM1, IFRD1, IGFBP2, VIM, PTPRJ, ECM1, GSTP1, C4BPA, C4BPB, SERPINA5, TTHY, CPN2 and APOH; THBS1, IGFBP3, C5, CLU, IGFBP2, ECM1, ICAM1, SERPINC1, CFI, ADIPO, PTPRJ, KLKB1, C6, PPBP, and C4BPA; TTHY, ITIH4, C4BPB, CLU, SEPP1, KLKB1, C1R, PTPRJ, ECM1, ADIPO, SERPINA5, VIM, ICAM1, IGFBP2, C4BPA, C5, PPBP, THBS1, C1S, C6, SERPINC1, APOH, and IGFBP3; THBS1, IGFBP2, C5, CLU, ECM1, ICAM1, TTHY, C4BPA, IFRD1, KLKB1, C1R and C4BPB; THBS1, IGFBP2, IGFBP3, C5, CLU, ECM1, ICAM1, TTHY, C4BPA, C7, SERPINC1, PPBP, SERPINA5, VIM and LDHB; TTHY, C4BPB, CLU, C1R, ECM1, SERPINA5, LDHB, COL4A2, SERPINC1, VIM, ICAM1, IGFBP2, C4BPA, C5, PPBP, C1S, LRG1, APOH, C7 and IGFBP3; THBS1, HSPG2, TTHY, IFRD1, IGFBP2, ECM1, ICAM1, SFTPB, SPARC, C4BPA, KLKB1, SERPINA5, C5 and COL4A2; TTHY, KLKB1, PTPRJ, ECM1, SFTPB, SERPINA5, COL4A2, ITIH2, ICAM1, IGFBP2, CPN2, C4BPA, C5, SPARC, HSPG2 and THBS1; PPBP, HRG, PKM2, LRG1, AGT, ICAM1, PDCD4, C7, C5, ITIH4, CTSD, IGFBP3, C4BPA, LDHB and APOC1; PKM2, HRG, AGT, APOC1, PDCD4, THBS1, C5, LDHB, PPBP, ITIH4, CFH, C4BPA, C1S, LRG1, C7, ICAM1, IGFBP3, CTSD and SOD3; PKM2, TXN, HRG, AGT, APOC1, CFH, PDCD4, THBS1, C5, LDHB, PPBP, ITIH4, FSTL1, C4BPA, C1S, LRG1, C7, ICAM1, IGFBP3 and CTSD; PPBP, HRG, LRG1, PDCD4, AGT, MBL2, PKM2, ICAM1, THBS1, C5, ITIH4, C7, APOC1 and CFH; LRG1, SEPP1, IGFBP3, CLU, SERPINC1, C1R, P4HB, CDH11, FCGBP, CPN2 and BTD; and P4HB, CORO1C, FCGBP, MMSA, SERPINC1, SEPP1, LRG1, C1R, BTD, CPN2, IGFBP3, CLU and CAP1.

In an exemplary embodiment, the combination of biomarkers or the biomarker panel according to the present application may further include CA19-9, previously known as a marker for diagnosing pancreatic cancer.

For the construction of the biomarker panel according to the present application, a combination of biomarkers showing an optimum effect for the use according to the present application may be selected and used.

Thus, in another aspect, the present application relates to a method for constructing a biomarker panel which selects the combination of markers to be included in the biomarker panel using a machine learning method, for example, using expression level data of the plurality of biomarkers as shown in Table 1.

Machine learning basically analyzes data using algorithms, learns through analysis, and makes a decision or prediction based on what is learned. Although machine learning aims to learn a method of performing a task by teaching the computer itself through a large amount of data and algorithms, it is very important to select an algorithm and optimize a parameter used in each algorithm in order to develop a predictive and diagnostic model with excellent performance.

In an exemplary embodiment, in the marker according to the present application, a plurality of two or more markers are used as a panel. The present application constructed a marker panel for diagnosing pancreatic cancer using a machine learning method, and particularly constructs a multi-marker panel through deep learning, a support vector machine (SVM), and logistic regression. Deep learning, one of the machine learning methods, may provide better performance by overcoming the limitations of existing linear models, extracting features from high-dimensional complex data, or extracting and understanding patterns.

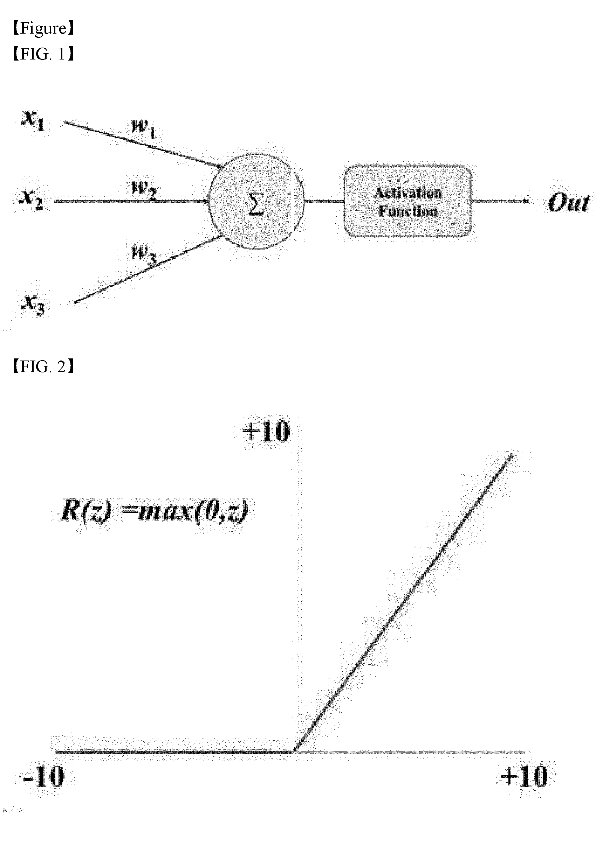

A basic unit that constitutes a network by the deep learning algorithm is the perceptron shown in FIG. 1, and this perceptron includes an input value, a weighted value, and an activation function. A basic structure of the perceptron allows a product of the input value (x) and the weighted value to be input as an activation function, and this activation function generally plays a role of delivering a value to the next layer when the value is a predetermined value or more, or not delivering the value to the next layer when the value is a predetermined value or less. Due to the activation function with such characteristics, it is called deep learning or artificial neural network because it is similar to the structure of human neurons. As the input value of the perceptron, data that a user wants to analyze (in the case of the present invention, the value of the peak area ratio of the endogenous peptide and the SIS peptide (internal standard material) by MRM-MS) is used, and as the weighted value, a value determined when learning the deep learning network for each characteristic (variable, feature) is used. Further, the activation function is a function which receives the product of an input value and a weighted value as an input value and determines which value to deliver when delivering the input value to the next layer. An artificial neural network, that is, a deep learning network is obtained by connecting a plurality of perceptions to constitute one building block and connecting a plurality of these building blocks. This building block becomes a layer commonly referred to in deep learning, and the perceptron constructed in each layer becomes a node or neuron.

The basic structure of a deep learning network includes an input layer, a hidden layer, and an output layer, and the deep learning has a feature that a sufficiently large number of hidden layers can be constituted. In an exemplary embodiment, as the deep learning network used in the present application, a feed-forward neural network in the basic form of deep learning was used. The activation function, which receives the product of the input value and the weighted value as an input, plays an important role in determining the performance of the model when a deep learning network is learned. Types of activation functions which are often used in deep learning include a sigmoid function, a rectified linear unit (ReLU), hyperbolic tangent (TanH), and the like, and in an exemplary embodiment, learning was performed and a model was constructed using the ReLU function in FIG. 2.

In the model constructed using deep learning, two hidden layers included 20 nodes, and ReLU was used as the activation function. A deep learning network constructed in this manner receives high-dimensional proteomics analysis data (in the case of the present invention, quantitative analysis results in 1008 blood samples for 68 peptides) to proceed with learning, and may determine the weighted value of each node and the combination of proteins constituting the model at the time of learning.

SVM is also a type of machine learning and is usually used for classification and regression analysis. When a set of data that belongs to one of two groups is given, SVM creates a linear classification model that determines which group the new data belongs to based on the given data set. As a result, SVM is an algorithm which finds the optimal hyperplane (decision boundary) capable of separating two different groups of data. SVM may be used for non-linear classification as well as linear classification, a task of mapping the given data to a high-dimensional space is required, but a kernel trick is also used to make this efficient. The kernel trick usually used in SVM includes a Gaussian radial basis function (RBF), polynomial, and sigmoid, and in the present invention, a model was constructed using a RBF kernel.

When the RBF kernel of SVM is used, it is essential to establish two parameters, Cost and Gamma. Cost is a parameter to how much error is allowed in the SVM model. That is, Cost determines how many data samples are allowed to be placed in other groups, and the smaller the cost value, the more allowed, and the larger the cost value, the less allowed. A model with a low cost finds a general hyperplane, whereas a model with a high cost finds a hyperplane in which two classes to be classified are more perfectly classified. Therefore, overfitting or underfitting may occur according to the Cost. Gamma is a parameter related to the above SVM kernel, and as a value which adjusts the standard deviation of the Gaussian function, the larger the value, the smaller the standard deviation. As the gamma is adjusted, the distance of influence of each piece of data varies, and when the gamma is small, the points far from the hyperplane are considered for classification, and when the gamma is large, only the points close to the hyperplane are considered for calculation. Since the gamma value may also be overfitted and underfitted as with the cost value, appropriate parameters need to be tuned according to the model.

Logistic regression is an algorithm which uses regression to predict the probability that data will belong to a group with a value between 0 and 1, and classify the data into that which belongs to a group with higher probability according to the probability. Logistic regression is a form in which odds are subjected to natural log (logit transformation) transformation for a general linear model, and as in the general linear model, a linear model is also obtained from the logistic regression.

For the construction of the biomarker panel according to the present application, expression level data of a plurality of biomarkers is obtained using mass spectrometry in blood isolated from a subject, the plurality of biomarkers are biomarkers in which the expression level in patients with pancreatic cancer is increased compared to an expression level of a control, and the control is normal and benign pancreatic tumor.

In an exemplary embodiment, for the expression data used in the method according to the present application, the MRM-MS quantitative analysis value, which is a mass spectrometry method, is used as an input value in a deep learning model. This input value is multiplied by a weighted value corresponding to each protein (variable) of a model for which learning has already been completed, and then delivered to the next layer via the activation function, and this process is performed while passing through two hidden layers. Thereafter, a final predicted value comes out from the final output layer, and it is possible to distinguish between a patient with pancreatic cancer and a normal person through this value.

The mass spectrometry used for the method according to the present application may include tandem mass spectrometry, ion trap mass spectrometry, triple quadrupole mass spectrometry, hybrid ion trap/quadrupole mass spectrometry and/or time-of-flight mass spectrometry. A mass spectrometry mode used in this case may be, for example, selected reaction monitoring (SRM) or multiple reaction monitoring (MRM).

In an exemplary embodiment according to the present application, in particular, a method of inducing ions to a quadrupole anode consisting of four electrode columns to perform an analysis according to the mass/charge (m/z) ratio, for example, a multiple reaction monitoring (MRM) mode using a triple quadrupole (Q1, Q2, Q3) mass spectrometer is used. The principle of MRM mass spectrometry is to hydrolyze all selected target proteins with peptides, and then select a peptide (precursor ion, MS1) with a mass to charge (m/z) specific to each target protein. When this specific peptide collides (Quadruple 2, Q2), a fragment with a specific mass (fragmentation ion, MS2) with a characteristic m/z is selected from the generated fragments. A pair of precursor fragments obtained from MS1/MS2 is named a specific transition of a target protein (specific mass fingerprint of the target protein), and when these transitions are measured for all target proteins (300 proteins or more), the amount of all target proteins in the sample may be relatively or absolutely quantified at the same time. For relative or absolute quantification, a SIS (isotopically substituted identical amino acid order) peptide is used as a standard material, and since the amount of input standard material (SIS peptide) of a sample to be measured is known, it is a principle capable of proportionally calculating the amount of target peptide. A transition that has passed through MS2 is converted to a digital signal at a detector and converted to a peak chromatogram, and relative and absolute quantitative analysis may be performed by calculating a peak area. Based on this principle, MRM can selectively detect and quantify only an analysis target to be targeted with high sensitivity.

A peptide to be analyzed in MRM analysis is a partial sequence used for MRM analysis in the entire protein sequence of a target to be analyzed, and it is possible to select an optimum target peptide which satisfies predetermined conditions in the entire sequence of proteins, such as conditions, for example, representativeness of a protein to be analyzed, the presence and absence of modification, and length suitable for MRM analysis. For example, peptides with an amino acid length of 6 to 30 are selected. When the length is much shorter than the above range, selectivity deteriorates, and when the length is much longer than the above range, sensitivity deteriorates. Furthermore, methionine, cysteine, and tryptophan are easily chemically modified like oxidation, and excluded at the time of selection. In an exemplary embodiment according to the present application, target peptides used when the biomarker according to the present application is analyzed by MRM are described in Table 2.

Further, in MRM, an internal standard material corresponding to the target peptide is used. Although the internal standard material has the same amino acid sequence as the above-described target peptide, the mass of one or more amino acids constituting the internal standard material is different from the amino acid mass of the target peptide. However, the internal standard material has the same hydrophobicity as the amino acid sequence, and thus is eluted at the same retention time as the target peptide. Through this, it is possible to confirm whether the target peptide is derived from the actual protein to be analyzed.

In still another aspect, the present application also relates to a biomarker panel or composition or kit for diagnosing pancreatic cancer, including a material or reagent capable of detecting each biomarker of a marker or combination of markers disclosed in the present application.

In the present application, detection includes quantitative and/or qualitative analysis, and includes the detection of presence and absence and the detection of an expression level, and these methods are known in the art, and a person skilled in the art may select an appropriate method for the implementation of the present application.

The detection of such a marker according to the present application may be based on the functional and/or antigenic features of the marker.

In an exemplary embodiment, the marker according to the present application may be detected using a nucleic acid which detects the activity or function of the marker, or encodes a protein, particularly, a material which specifically interacts at the mRNA level and/or protein level.

From this aspect, the detection reagent included in the composition according to the present application is a reagent capable of detecting the marker according to the present application by quantitative or qualitative analysis by various methods at the protein or nucleic acid level.

Various methods for qualitatively or quantitatively detecting a known protein or nucleic acid may be used for quantitative or qualitative analysis of the marker according to the present application.

As the qualitative or quantitative detection method at the protein level, it is possible to use, for example, a method using western blot, ELISA, radioimmunoassay, immunodiffusion technique, immunoelectrophoresis, tissue immunostaining, immunoprecipitation assay, complement fixation assay, binding to an antibody labeled in a solution/suspension, mass spectrometer, protein array using an antibody, or the like.

Alternatively, as the quantitative or qualitative detection method as the nucleic acid level, it is possible to use a method using nucleic acid transcription and amplification method, eTag system, a system based on labeled beads, an array system such as nucleic acid array, and the like.

These methods are known, and for example, chip-based capillary electrophoresis: Colyer et al. 1997. J Chromatogr A. 781(1-2):271-6; mass spectroscopy: Petricoin et al. 2002. Lancet 359: 572-77; eTag systems: Chan-Hui et al. 2004. Clinical Immunology 111:162-174; microparticle-enhanced nephelometric immunoassay: Montagne et al. 1992. Eur J Clin Chem Clin Biochem. 30:217-22, and the like may be referenced.

In an exemplary embodiment according to the present application, a marker may be detected using mass spectrometry, in which a protein or peptide may be isolated from a specimen, and then analyzed by the method described in the embodiments of the present application, and further, for example, (Kim, et al. 2010 J Proteome Res. 9: 689-99; Anderson, L et al. 2006. Mol Cell Proteomics 5: 573-88.) may be referenced. In one exemplary embodiment, a multiple reaction monitoring (MRM) technology using, for example, Triple Quadrupole LC-MS/MS, QTRAP, and the like is used. For MRM, those previously mentioned may be referenced.

In another exemplary embodiment, a binding preparation that specifically binds to each protein or mRNA derived from a gene encoding the protein, or an array including the binding preparation is used.

In still another exemplary embodiment, a sandwich-type immunoassay such as enzyme linked immunosorbent assay (ELISA) and radioimmunoassay (RIA) may be used. These methods may qualitatively or quantitatively detect a protein by adding a biological sample to a first antibody bound to a solid substrate, for example, glass, plastic (for example, polystyrene), polysaccharide, beads made of nylon or nitrocellulose, a membrane, a slide or a microtiter plate, and then labeling the sample with a labeling material capable of direct or indirect detection, for example, a radioactive material such as $^3$H or $^{125}$I, a fluorescent material, a chemical luminescent material, a hapten, biotin, digoxigenin, and the like, or by binding to an antibody conjugated with an enzyme such as horseradish peroxidase, alkaline phosphatase, and malate dehydrogenase, which can develop color or emit light by action with the substrate.

In yet another exemplary embodiment, it is possible to use immunoelectrophoresis such as Ouchterlony plate, western blot, crossed IE, rocket IE, fused rocket IE, and affinity IE, which are capable of easily detecting a marker by antigen-antibody binding. The immunoanalysis or immunostaining method is described in Enzyme Immunoassay, E. T. Maggio, ed., CRC Press, Boca Raton, Florida, 1980; Gaastra, W., Enzyme-linked immunosorbent assay(ELISA), in Methods in Molecular Biology, Vol. 1, Walker, J.M. ed., Humana Press, NJ, 1984, and the like. It is possible to diagnose whether a disease occurs by analyzing the intensity of a final signal by the above-described immunoassay process, that is, by performing a signal comparison with a normal sample.

Reagents or materials used in such methods are publicly known, and for example, antibodies, substrates, nucleic acids or peptide aptamers that specifically bind to the marker, or receptors, or ligands, or cofactors, and the like that specifically interact with the marker may be used. Reagents or materials that specifically interact with or bind to the marker may be used in a chip method or with nanoparticles.

The marker of the present application may be detected quantitatively and/or qualitatively using various known methods at the nucleic acid level, particularly, the mRNA level.

As the qualitative or quantitative detection method at the nucleic acid level, for example, it is possible to use a method using reverse transcription-polymerase chain reaction (RT-PCR)/polymerase chain reaction, competitive RT-PCR, real-time RT-PCR, nuclease protection analysis (NPA), for example, RNase, S1 nuclease analysis, *in situ* cross method, DNA microarray or chip, or northern blot, and the like for detection at the mRNA level or detection of the expression level or pattern, these analysis methods are known, the method may be performed using a commercially available kit, and a person skilled in the art will select those appropriate for the implementation of the present application. For example, northern blot has an advantage in that the size of transcriptome present in cells can be known and various probes can be used, the NPA is useful for multi-marker analysis, the *in situ* cross method easily locates the position of a transcriptome such as mRNA in cells or tissues, and the reverse transcription-polymerase chain reaction is useful for detecting a small amount of sample. Further, it is possible to use a binding preparation that specifically binds to a nucleic acid such as mRNA or cRNA derived from a gene encoding a biomarker protein, or an array including the binding preparation.

Reagents or materials used in the method for detecting a biomarker at the nucleic acid level are known, and for example, in a method for determining the presence or absence of mRNA and measuring the amount of mRNA by RT-PCR, the detection reagent includes, for example, a polymerase, a probe and/or primer pair specific to the mRNA of the marker of the present application. A primer or probe refers to a nucleic acid having a free 3' hydroxyl group that can bind complementarily to a template and allow a reverse transcriptase or DNA polymerization enzyme to initiate the replication of the template. The detection reagents used in the present application may be labeled with such a color development, luminescent or fluorescent material as described above for signal detection. In an exemplary embodiment, northern blot or reverse transcription PCR (polymerase chain reaction) is used for mRNA detection. The latter case isolates RNA of a specimen, particularly, mRNA, then synthesizes cDNA therefrom, and then detects a specific gene in the specimen using a specific primer, or a combination of a primer and a probe, and is a method capable of determining the presence/absence of or the expression level of the specific gene. This method is described, for example, in (Han, H. et al, 2002. Cancer Res. 62: 2890-6).

A detection reagent included in the biomarker panel or composition according to the present application may be labeled directly or indirectly in the form of a sandwich for detection, depending on a specific method used for detection. In the case of a direct labeling method, a serum sample used for an array or the like is labeled with a fluorescent label such as Cy3 or Cy5. **In** the case of the sandwich, an unlabeled serum sample is first reacted with an array to which a detection reagent is attached for binding, and then a target protein is bound to a labeled detection antibody for detection. The sandwich method may enhance sensitivity and specificity, thereby enabling detection up to the pg/mL level. **In** addition, a radioactive material, a color development material, magnetic particles, high-density electron particles, and the like may be used as a labeling material. A scanning confocal microscope may be used for fluorescence luminosity, and may be available, for example, from Affymetrix, Inc., Agilent Technologies, Inc., and the like.

The biomarker panel or composition according to the present application may further include one or more additional components required for binding analysis, and may further include, for example, a binding buffer, a reagent required for sample preparation, a blood sampling syringe or a negative and positive control.

The biomarker panels or compositions according to the present application including various detection reagents as described above may be provided for ELISA analysis, dip stick rapid kit analysis, an MRM analysis kit, a microarray, gene amplification, immunoassay, and the like depending on the analysis aspect, and an appropriate detection reagent may be selected according to the analytical aspect.

**In** an exemplary embodiment, ELISA or a dip stick rapid kit is used, and in this case, an antibody which recognizes one or more markers according to the present application may be provided while adhering to a substrate, for example, a well of a multi-well plate or the surface of a glass slide, or nitrocellulose. The dip stick is a technology widely used in the point of care test (POCT) field, and detects a marker by a method of developing a color when one or more antibodies which recognize the biomarker according to the present application are bound to a substrate such as nitrocellulose, and the same is brought into contact with a sample such as serum, for example, when one end of the dip stick is immersed in a serum sample, the sample moves to the substrate by a capillary phenomenon to bind to the antibody in the substrate.

In yet another exemplary embodiment, a peptide-based MRM kit is provided, and the MRM method is as described above. The MRM method uses a peptide which selectively recognizes a specific protein, and may detect a marker from a biological sample more stably than existing methods using an antibody which is sensitive to the environment such as temperature and humidity. For example, the peptides described above may be used, and one or more peptides may be used for one marker.

In yet another exemplary embodiment, a peptide may be provided in the form of an array or chip including microarrays. A detection reagent may be attached to the surface of a substrate such as glass or nitrocellulose, and for the array preparation technology, for example, Schena et al., 1996, Proc Natl Acad Sci USA. 93(20):10614-9; Schena et al., 1995, Science 270(5235):467-70; and U.S. Pat. Nos. 5,599,695, 5,556,752 or 5,631,734 may be referenced. A detection reagent which may be attached to an array includes, for example, antibodies, antibody fragments, aptamers, avidity multimers or peptidomimetics capable of specifically binding to one protein.

In yet another aspect, the present application relates to a kit or system for diagnosing pancreatic cancer, including a detection reagent of a biomarker. A detection reagent and a method of using the detection reagent are as described above. A reagent capable of detecting the marker of the present application may be present while being individually dispensed in a partitioned container, and in this sense, the present application also relates to a device/tool which includes the marker detection reagent of the present application in a partitioned manner. Further, the kit may further include instructions for use.

In yet another aspect, in order to provide information necessary for the diagnosis of pancreatic cancer, the present application relates to a method for detecting a biomarker for diagnosing pancreatic cancer *in vitro,* the method including: measuring the expression level of a biomarker panel including a combination of the biomarkers disclosed in Tables 5, 8, and 9-1 and 9-2, and the like from a biological sample such as blood isolated from a subject including a human or a mammal other than a human; and correlating with pancreatic cancer by comparing the measurement result with a measurement result corresponding to the corresponding marker of a control sample.

In the present application, the biological sample includes all solid or liquid samples obtained from the human body or a mammal, for example, tissue derived from a specific organ, and urine, saliva, whole blood, plasma or serum samples, but is not limited thereto. In an exemplary embodiment, the marker protein of the present invention is included in a tissue or blood sample, particularly blood.

In the method according to the present application, the control is normal and/or benign pancreatic tumors.

In the method according to the present application, each biomarker panel further includes CA19-9.

In the correlating with pancreatic cancer in the method according to the present application, as a result of the measurement of the subject, when the expression level increases or decreases compared to the expression level measured in the control, the subject is determined to have pancreatic cancer. For the increase or decrease in expression of the biomarker according to the present application compared to the control, those disclosed in the present application may be referenced.

In the method according to the present application, the control is a sample derived from a subject having normal and/or benign pancreatic tumors.

In the method according to the present application, for a method of measuring the expression level of each biomarker in the measuring of the expression level, those previously mentioned may be referenced.

In an exemplary embodiment, the method is performed by mass spectrometry including tandem mass spectrometry, ion trap mass spectrometry, triple quadrupole mass spectrometry, hybrid ion trap/quadrupole mass spectrometry or time-of-flight mass spectrometry. A mode used for the mass spectrometry is selected reaction monitoring (SRM) or multiple reaction monitoring (MRM), particularly MRM.

In an exemplary embodiment, MRM analysis is used, and the peptides used for the analysis are as shown in Table 2.

Hereinafter, the following Examples for helping the understanding of the present invention will be suggested. However, the following Examples are provided only to more easily understand the present invention, and the present invention is not limited to the following Examples.

## Examples

### Example 1. Pretreatment of blood sample

### <1-1> Preparation of plasma sample

In order to discover an effective protein biomarker capable of diagnosing pancreatic cancer, plasma samples of patients with pancreatic cancer were collected from a total of five hospitals: Asan Medical Center, National Cancer Center, Samsung Medical Center, Seoul National University Hospital, and Yonsei Severance Hospital. All plasma samples were collected in EDTA-coated tubes, and frozen and stored immediately after being aliquoted. Hemolytic samples were excluded, and for plasma samples from patients with pancreatic cancer, serum samples of patients who underwent surgery or chemotherapy were taken.

### 1-2. Plasma protein depletion process

In order to discover a biomarker present at a low concentration in blood, 40 μL of blood was taken for each individual sample, and was subjected to a depletion process of removing 7 types (albumin, IgG, IgA, haptoglobin, transferrin, antitrypsin, and fibrinogen) present in the highest proportion in the blood. By removing 7 highly abundant proteins, about 88 to 92% of the total proteins in the blood were removed, and the proteins present in a small amount were analyzed using only the remaining 8 to 12% of the proteins.

### 1-3. Plasma protein, peptidization process

A plasma sample obtained after the depletion process was concentrated (w/ 3K filter), and then the protein concentration was quantified by bicinchoninic acid (BCA) assay method. 200 μg of the plasma sample was taken, treated with a final concentration of 6 M urea/20 mM DTT (Tris pH 8.0), and then incubated at 37°C for 60 minutes. The sample was treated with a final concentration of 50 mM IAA, and then incubated at room temperature for 30 minutes. The sample was treated with 100 mM Tris pH 8.0, such that the concentration of urea was 0.6 M or less. The sample was treated with trypsin such that a ratio of trypsin and plasma was 1:50, and then incubated at 37°C for 16 hours. After the sample was treated with a formic acid solution such that the final concentration was 5%, desalting was performed.

### 1-4. Plasma protein, desalting process

An OASIS column was activated by flowing 1 mL of 60% ACN / 0.1% formic acid three times, and equilibration was performed by flowing 1 mL of 0.1% formic acid in the OASIS column five times. A peptide sample was introduced and washed by flowing 1 mL of 0.1% formic acid five times. Peptide elution was performed by treating the sample with 1 mL of 40% ACN / 0.1% formic acid and 1 mL of 60% ACN / 0.1% formic acid. The sample was frozen at -70°C for 1 hour or more and dried by speed-vac. The dried peptide sample was dissolved in 50 μL of a Sol A buffer (3% ACN / 0.1% formic acid) and then centrifuged at 15,000 rpm for 60 minutes, and analysis was performed by transferring only 40 μl of the centrifuged sample to a vial.

### 1-5. MRM analysis process

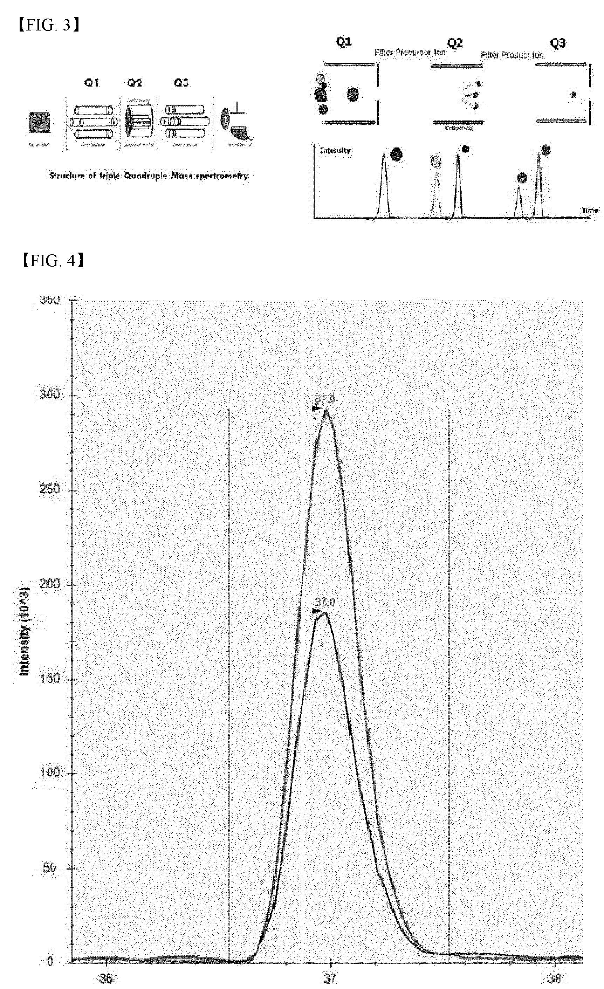

Referring to FIG. 3, Quadruple 1 (Q1) serves as a filter capable of allowing only a specific Q1 m/z to pass through. A precursor ion which has passed through the Q1 filter is fragmented by electrical energy in Quadruple 2 (collision cell) and decomposed into product ions. For this product ion, only a specific product ion can pass through Quadruple 3 (Q3) serving as a filter as in Quadruple 1 (Q1). The ion that has passed through Quadruple 3 (Q3) is converted to a digital signal at a detector and is displayed on a peak chromatogram, and relative and absolute quantitative analysis may be performed by analyzing the area of the peak.

**Example 2. Selection of biomarker candidate group**

**2-1. Clinical samples used in study**

In order to select a detectable and quantifiable target from mass spectrometry of target proteins selected as a marker candidate group, a total of 134 samples including 50 cases of pancreatic cancer, 34 cases of pancreatic benign disease, and 50 cases of normal persons from Seoul National University Hospital were used in the discovery and verification steps. In order to finally discover an effective protein biomarker capable of diagnosing pancreatic cancer based on the marker candidate proteins selected in the discovery & verification steps, plasma samples of patients with pancreatic cancer obtained from a total of five hospitals of Asan Medical Center, National Cancer Center, Samsung Medical Center, Seoul National University Hospital, and Yonsei Severance Hospital were used, and a total of 401 samples were prepared. Other carcinoma samples were also prepared as a sample group for comparison with other cancers other than pancreatic cancer. Other carcinomas totaled 149 cases including 52 cases of breast cancer, 45 cases of colorectal cancer, and 52 cases of thyroid cancer. Samples for benign tumors of the pancreas and other benign diseases such as pancreatitis and cholecystitis were also prepared, and the number of samples totaled 109 cases. A total of 349 plasma samples of normal patients were prepared at Seoul National University Hospital.

**2-2. Selection of marker candidate**

In order to select a marker candidate group related to pancreatic cancer, a paper on pancreatic cancer research using 17 proteomics techniques was first referenced, and here, a total of 819 proteins were selected as marker candidates. 8 common databases were obtained from ONCOMINE, and 8,145 genes were retrieved as candidates. 753 genes were screened in Metacore Pathway Studio, and a total of 2226 genes were confirmed through 3 differential expressed gene (DEG) papers. A total of 508 proteins were set as marker candidates through the aforementioned data-mining process. A marker candidate group selection process using a tissue microarray (hereinafter referred to as TMA) was also performed. TMA was performed using 104 cases of pancreatic cancer, 50 cases of IPMN, and 17 cases of normal persons, 22,785 mRNAs specifically expressed in pancreatic cancer and specifically expressed in plasma and serum were selected, and 456 proteins were finally selected at the protein level. A total of 1,000 candidates, including 22 generally known cancer-related marker proteins and 14 mutant proteins, were selected as a marker candidate group for the diagnosis of pancreatic cancer.

**2-3. Selection of selectable target candidate group**

As a result of filtering using the MSMS library of the National Institute of Standards and Technology (NIST) to select candidates detected by a mass spectrometer from 1000 candidate proteins, 907 were selected. When 907 proteins were selected, the proteins were selected using UniprotKB, such that 1 to 20 peptides belonged to each protein, and transitions for each peptide were generated using the Skyline program. For the number of amino acids in the peptide, peptides having a length of 7 to 24 amino acids were used. Actually, in order to select only a target which can be detected by mass spectrometry equipment, as a result of selecting only peptides with proper signal detection by MRM analysis using pooling samples prepared from 6 normal subjects and 6 pancreatic cancer subjects, 225 types of proteins were detected in actual plasma samples.

**2-4. Semi-quantitative MRM analysis and selection of final marker candidates**

225 proteins selected as detectable by mass spectrometer were subjected to relative quantification using a sample consisting of 134 pancreatic cancer, normal, and pancreatic benign diseases for the candidate group (FIG. 4). For the purpose of securing quantification, the concentration is normalized with the peptide area value of the corresponding peptide at the time of all MRM analyses using a known specific peptide, a peptide corresponding to this is called an internal standard material, and the peptide is a peptide having an amino acid labeled with a radioisotope. A sample was prepared by spiking 50 fmol of an internal standard material β-galactosidae [GDFQFNISR (13C15N)] into each sample, and the peak area values of all target peptides from the MRM analysis were normalized with the peak area values of the corresponding internal standard material.

As a result of the analysis, it was found that 205 proteins and 316 peptides showed AUC> 0.6 or higher performance in normal, pancreatic cancer and pancreatic benign disease, and had an ability to distinguish pancreatic cancer. During detection in a mass spectrometer from thus selected 205 proteins and 316 peptides, stable-isotope labeled standard (SIS) peptides were synthesized for the final 176 proteins and 217 peptides by removing peptides with a signal interference phenomenon. This synthetic SIS peptide is a peptide labeled with a radioisotope in lysine (13C615N2, 8 Da mas shift) or arginine (13C615N4, 10Da mass shift) at the C-termini. This SIS peptide has all the same properties as an endogenous peptide already present in the plasma sample, except that the SIS peptide has a mass value different from the endogenous peptide, and the amount of endogenous peptide is measured through a peak area ratio value of endogenous peptide and SIS peptide using the SIS peptide as an internal standard material.

MRM relative quantitative analysis was performed using the same samples as before, including 50 cases of pancreatic cancer, 34 cases of pancreatic benign disease, and 50 cases of normal persons using 176 proteins and 217 peptides from which the SIS peptide was synthesized. The analysis was repeated 3 times for each sample, and as a result of the analysis, 65 proteins and 79 peptides were quantified to be expressed differently in pancreatic cancer, normal and pancreatic benign disease. In addition, 54 proteins and 68 peptides showed AUC> 0.6 distinguishing performance in normal and pancreatic cancer by MRM relative quantification by comparing the concentrations of the SIS peptide and the endogenous peptide, showing an ability to distinguish pancreatic cancer. Furthermore, an automated detection of inaccurate and imprecise transitions (AuDIT) tool built into Skyline was used to determine the presence or absence of the interference phenomenon of the peak signals. Based on a coefficient of variation (CV) value of each target peptide for the results of three repeated analyses, an analysis candidate group with reproducibility and quantitativeness of 20% or less were selected. As a result, 54 proteins and 68 peptides analyzed without any interference phenomenon were selected as a final marker candidate group (Table 2).

[Table 2]

| Gene Symbol | Peptide Sequence | Gene Symbol | Peptide Sequence |
|---|---|---|---|
| ADIPOQ | GDIGETGVPGAEGPR | ICAM1 | LLGIETPLPK |
| AGT | DPTFIPAPIQAK | | VTLNGVPAQPLGPR |
| ALDH6A1 (MMSA) | QGIQFYTQLK | IFRD1 | GLIDLTLDK |
| APOC1 | EFGNTLEDK | IGFBP2 | LIQGAPTIR |
| | EWFSETFQK | | ALAQCAPPPAVCAELVR |
| APOH | VCPFAGILENGAVR | IGFBP3 | YGQPLPGYTTK |
| BTD | ILSGDPYCEK | ITIH2 | IQPSGGTNINEALLR |
| | LSSGLVTAALYGR | ITIH4 | LALDNGGLAR |
| C1R | DYFIATCK | | AGFSWIEVTFK |
| C1S | TNFDNDIALVR | KLKB1 | DSVTGTLPK |
| C4BPA | TWYPEVPK | LDHB | IVVVTAGVR |
| | LSLEIEQLELQR | | GLTSVINQK |
| C4BPB | ALLAFQESK | LRG1 | DLLLPQPDLR |
| C5 | NADYSYSVWK | | VAAGAFQGLR |
| | GGSASTWLTAFALR | MBL2 | FQASVATPR |
| C6 | TLNICEVGTIR | P4HB | ALAPEYAK |
| C7 | VLFYVDSEK | PDCD4 | GDSVSDSGSDALR |
| CAP1 | EPAVLELEGK | PKM2 | CDENILWLDYK |
| CDH11 | VLDVNDNAPK | PPBP | NIQSLEVIGK |
| CFH | GEWVALNPLR | | ICLDPDAPR |
| | TGESVEFVCK | PROS1 | NNLELSTPLK |
| CFI | VFSLQWGEVK | PTPRJ | DTEVLLVGLEPGTR |
| CLU | TLLSNLEEAK | SEPP1 | CINQLLCK |
| | ASSIIDELFQDR | SERPINA5 (IPSP) | GFQQLLQELNQPR |
| COL4A2 | GLPGEVLGAQPGPR | SERPINC1 | VAEGTQVLELPFK |
| CORO1C | CDLISIPK | | IEDGFSLK |
| CPN2 | LSNNALSGLPQGVFGK | SFTPB | FLEQECNVLPLK |
| CTSD | VSTLPAITLK | SOD3 | VTGVVLFR |
| ECM1 | ELLALIQLER | SPARC | NVLVTLYER |
| FCGBP | FAVLQENVAWGNR | THBS1 | GFLLLASLR |
| FSTL1 | LSFQEFLK | | TIVTTLQDSIR |
| GSTP1 | ASCLYGQLPK | TTHY | AADDTWEPFASGK |
| HRG | DGYLFQLLR | TXN | CMPTFQFFK |
| HSPG2 | SPAYTLVWTR | VIM | ILLAELEQLK |

**2-5. Large-scale individual sample analysis of multiple institutions**

To discover biomarkers for the diagnosis and prediction of pancreatic cancer through a blood test of 54 proteins and 68 peptides that can be stably analyzed in plasma samples of subjects with pancreatic cancer and normal persons by a mass spectrometer, a validation analysis was performed using large-scale samples collected from multiple institutions. The institutions are the five hospitals of Asan Medical Center, National Cancer Center, Samsung Medical Center, Seoul National University Hospital, and Yonsei Severance Hospital in total, and the total number of samples used in the verification step was 1008 plasma samples. For MRM analysis, 1008 samples were divided into 4 batches. 250 samples were assigned to each batch, and 4 different sample groups were assigned to belong in similar proportions. The final batch consists of 258 samples by further including 8 samples. In addition, in order to correct the deviation of the analysis, pooling samples were randomly placed in each batch and analyzed together as a positive control. As the equipment used for the analysis, an Agilent 1260-capillary LC for liquid chromatography was used, and a column of capillary RR 0.5 x 150 3.5 um was used for the separation of peptides. 5 $\mu$l of the sample was injected, and the flow rate was set to 20 uL/min. First, after the column was equilibrated with Sol A (95% distilled water, 5% acetonitrile and 0.1% formic acid by volume) for 10 minutes, the peptide was eluted by applying a concentration gradient by a method of adjusting the proportion of Sol B (95% acetonitrile and 0.1% formic acid) to 3% to 35% for 50 minutes and 80% for 10 minutes and lowering the proportion of Sol B to 5% for the final 5 minutes. The total LC analysis time used for the analysis is 70 minutes. Transitions for selected proteins were monitored in the MRM mode using an Agilent triple quadrupole 6490-QQQ instrument as a mass spectrometer. A 5 fmol beta-galactosidase peptide (GDFQFNISR [C13N15], 547.3646.4) spiked into each sample was also monitored simultaneously to correct a deviation between batches. The order of MRM analysis was randomized after blinding such that the patient group could not be confirmed by the experimenter, and the analysis was repeated three times per sample. For the peak area value for the marker candidate group obtained by this process, a peak area value of the endogenous peptide was normalized with a peak area value of the corresponding SIS peptide to prepare data. This data was used to develop a multi-marker panel using machine learning.

**2-6. Analysis performance of reverse calibration curve and MRM-MS**

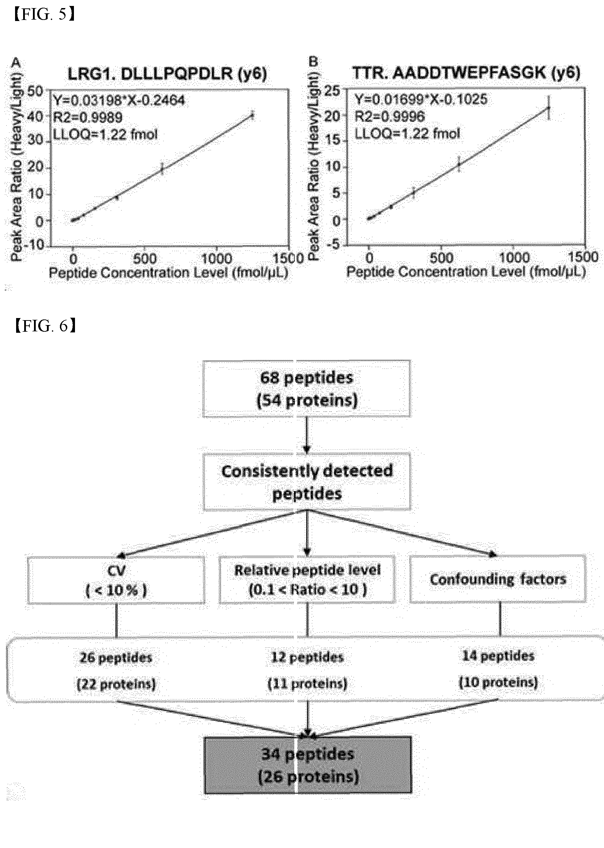

In order to evaluate that the analysis was stable when the MRM-MS analysis was performed using plasma samples, a process of confirming linearity by a reverse calibration curve was performed. A sample from which a curve would be drawn was prepared by continuously diluting the SIS peptide with a pooling sample prepared by pooling a serum sample at a ratio of 1/2. In order to draw a calibration curve, a sample with a total of 11 points was prepared, and the concentration values of the SIS peptide spiked at each point were set to 1250, 625.0, 312.5, 156.3, 78.1, 39.1, 19.5, 9.77, 4.88, 2.44, and 1.22 fmol. In addition, each point was set to include 3 to 4 transitions at the time of MRM analysis, and the analysis was performed repeatedly three times. A transition analyzed while showing the highest signal in the analysis was used for quantification, and the transition signal analyzed to be lowest was used as a background. The calibration curve was fitted through a linear regression method. The results are illustrated in FIG. 6. The lowest concentration point showing $R^2 > 0.998$ and CV < 20% through the calibration curve was set to the lower limit of quantitation (LLOQ), and it was confirmed that the analysis could be stabilized by confirming the linearity at the time of MRM analysis using a blood sample.

**Example 3. Construction of biomarker panel**

**3-1. Construction of biomarker panel using deep learning method**

**3-1-1. Data preprocessing**

Since it is important to be able to analyze the result with reproducibility during MRM-MA analysis by applying the result to actual clinical samples, a task of selecting marker candidate groups was performed before the selection of a biomarker panel through three criteria. First, the cutoff value of the coefficient of variation (CV) for three repeated analyses was set to 10%. Accordingly, 26 peptides and 22 proteins were excluded because they failed to meet the corresponding criteria. Second, a target peptide ratio (endogenous / SIS-peptide ratio) was used. Peptides whose peptide ratio was relatively quantified at 0.1 or more and 10 or less were used for analysis, and in this case, 12 peptides and 11 proteins were excluded. Third, a bias (confounding factor) according to each institution or hospital or analytical batch was removed, and 14 peptides and 10 proteins were considered to be the corresponding factors, and thus removed. As a result, 34 peptides and 25 proteins were selected as target candidate groups to be used for the final analysis.

**3-1-2. Development of biomarker panel**

The configuration of samples used to develop a multi-marker panel based on deep learning is shown in Table 3.

The training set consisted of 691 cases and the test set consisted of 317 cases to discover a marker for diagnosing pancreatic cancer, and a marker candidate group used to develop a multi-marker panel adopted 25 proteins and 34 peptides.

- Training set: 322 cases of pancreatic cancer (60 cases of Asan Medical Center, 102 cases of National Cancer Center, 82 cases of Samsung Medical Center, 40 cases of Seoul National University Hospital, and 38 cases of Yonsei Severance Hospital), 88 cases of pancreatic benign disease (33 cases of Asan Medical Center, 26 cases of Samsung Medical Center, 4 cases of Seoul National University Hospital, and 25 cases of Yonsei Severance Hospital), and 281 cases of normal persons
- Test set: 79 cases of pancreatic cancer (15 cases of Asan Medical Center, 26 cases of National Cancer Center, 19 cases of Samsung Medical Center, 10 cases of Seoul National University Hospital, and 9 cases of Yonsei Severance Hospital), 21 cases of pancreatic benign disease (14 cases of Asan Medical Center, 4 cases of Samsung Medical Center, 1 case of Seoul National University Hospital, and 2 cases of Yonsei Severance Hospital), and 68 cases of normal persons

[Table 3]

| Sample | Institution | Number of samples | | |
| --- | --- | --- | --- | --- |
| | | Total | Training set | Test set |
| Pancreatic cancer | | 401 | 322 | 79 |
| | Asan Medical Center | 75 | 60 | 15 |
| | National Cancer Center | 128 | 102 | 26 |
| | Samsung Medical Center | 101 | 82 | 19 |
| | Seoul National University Hospital | 50 | 40 | 10 |
| | Yonsei Severance Hospital | 47 | 38 | 9 |
| Other carcinomas | Seoul National University Hospital | 149 | 0 | 149 |
| Pancreatic benign disease | | 109 | 88 | 21 |
| | Asan Medical Center | 47 | 33 | 14 |
| | Samsung Medical Center | 30 | 26 | 4 |
| | Seoul National University Hospital | 5 | 4 | 1 |
| | Yonsei Severance Hospital | 27 | 25 | 2 |
| Normal | Seoul National University Hospital | 349 | 281 | 68 |
| Total | | 1,008 | 691 | 317 |

In order to discover a marker for diagnosing pancreatic cancer, Case consisted of pancreatic cancer, and Control consisted of normal + pancreatic benign disease + other carcinomas.

The present inventors attempted to construct a multi-marker panel through deep learning analysis, which is one of the machine learning analysis methods. A deep learning package used for the analysis was H2o (https://www.h2o.ai), and the present inventors attempted to construct a multi-marker panel using a feed-forward neural network, which is one of the deep learning algorithms provided by the package. In order to use the deep learning, it is fundamentally important to determine the activation function, the number of hidden layers, and the number of nodes in the hidden layer. After various optimization processes, the activation function used the following Function Formula (1) rectified linear unit (ReLU), the total number of hidden layers was set to 2 in total, and the number of nodes in each hidden layer was set to 20.

$$\text{Function Formula (1)}$$

$$ReLU\ (z)\ =\ max\ (0,\ z)$$

(z = input value, the value of the peak area ratio of the endogenous peptide and the SIS peptide (internal standard material) by MRM-MS in the case of the present invention).

In addition, when analysis is performed by applying deep learning, a process of optimizing parameters such as the number of learning repetitions (epochs), learning method, and Dropout is required. This process was also subjected to various parameter optimization processes and applied the most appropriate parameters. The Epoch was set to 400 times, the learning method was set to ADADELTA, and Dropout to prevent the overfitting of a model was applied to a hidden layer at a ratio of 0.5. As a result of generating a model which differentiates pancreatic cancer through comparative analysis of pancreatic cancer with normal + pancreatic benign disease + other carcinomas through deep learning by applying the above-mentioned and determined parameters, a multi-marker panel consisting of 14 peptides was constructed. The performance of this model showed an AUC of 0.9301, an accuracy of 89.8%, a sensitivity of 81.8%, and a specificity of 97.2% in the training set, and showed an AUC of 0.9427, an accuracy of 87.7%, a sensitivity of 91.1%, and a specificity of 86.5% in the test set, as shown in Table 4. It was able to distinguish pancreatic cancer with better performance than CA19-9 (a sensitivity of 79% and a specificity of 82%), which is currently used as a marker for diagnosing pancreatic cancer. 13 markers corresponding to the 14 peptides were TTHY, ITIH4, CLU, SEPP1, LRG1, KLKB1, C1R, SERPINA5, BTD, IGFBP2, C5, PROS1 and CFI, which are shown in detail in Table 5.

[Table 4]

| Set | metrics | | | | |
| --- | --- | --- | --- | --- | --- |
| | Accuracy | AUC | Sensitivity | Specificity | F1 score |
| Training Set | 0.8986 | 0.9301 | 0.8182 | 0.9722 | 0.8852 |
| Test Set | 0.8770 | 0.9472 | 0.9114 | 0.8655 | 0.7869 |

[Table 5]

| N. | Gene Symbol | Peptide Sequence |
|---|---|---|
| 1 | TTHY | AADDTWEPFASGK |
| 2 | ITIH4 | AGFSWIEVTFK |
| 3 | CLU | ASSIIDELFQDR |
| 4 | SEPP1 | CINQLLCK |
| 5 | LRG1 | DLLLPQPDLR |
| 6 | KLKB1 | DSVTGTLPK |
| 7 | C1R | DYFIATCK |
| 8 | IPSP | GFQQLLQELNQPR |
| 9 | BTD | ILSGDPYCEK |
| 10 | IGFBP2 | LIQGAPTIR |
| 11 | BTD | LSSGLVTAALYGR |
| 12 | C5 | NADYSYSVWK |
| 13 | PROS1 | NNLELSTPLK |
| 14 | CFI | VFSLQWGEVK |

**3-1-3. Performance of optimization of deep learning network**

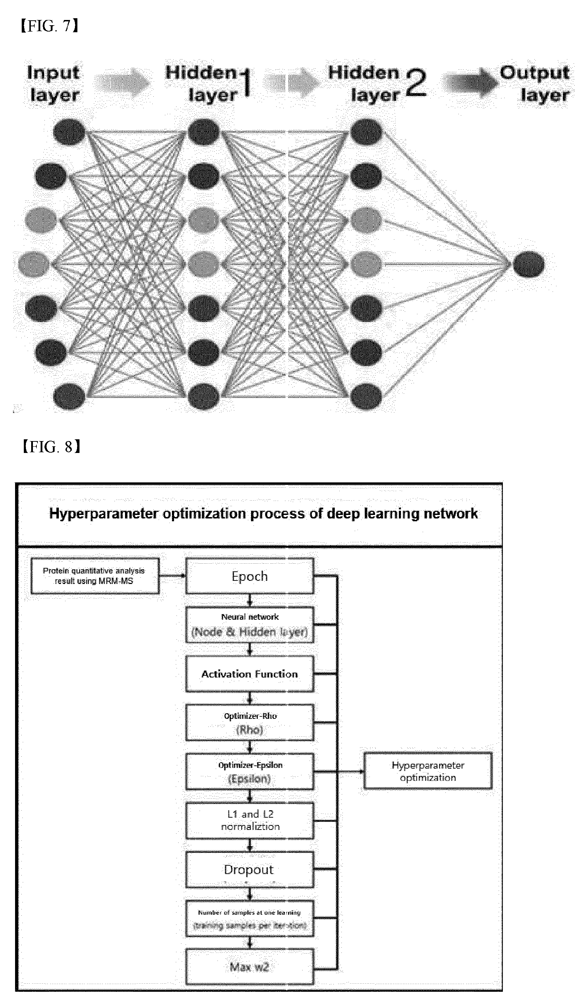

In order to optimize hyperparameters when deep learning was used, a deep learning network through the optimization process proceeded with learning, and in order to show the best performance, a process of optimizing each hyperparameter was performed (FIG. 7).

For deep learning, the hyperparameters to be optimized vary depending on the type of algorithm used and the analysis data, and in the present application, optimization was performed on 9 hyperparameters which are generally most importantly used when applying deep learning (FIG. 8).

(1) Activation function

The activation function is a function which is present in multiple hidden layers often referred to in deep learning, and a calculated value is delivered from the previous layer to the next layer through this function, and this activation function is used to have the non-linear characteristics of deep learning. Currently, the activation functions usually used in deep learning include hyperbolic tangent (tanh), ReLU, Maxout, and the like, and in this study, all three activation functions were tested. As a result of performing the test by keeping all the remaining hyperparameters the same and changing only the activation function, a network using ReLU as an activation function showed: Training Set AUC: 0.909, Test Set AUC: 0.9453, showing the best performance.

(2) Hidden layer, node and neural network (Node and Layer)

A deep learning network consists of an artificial neural network consisting of multiple hidden layers between an input layer and an output layer, and each layer consists of multiple nodes which receive input values. As a process of optimizing the number of these layers and the number of nodes is also required, an optimization process was performed by dividing the number of layers and the number of nodes in each layer by the number of cases such as 100, 200, 50-50,100-100, 35-35-35, 70-70-70, 25-25-25-25, and 50-50-50-50. Even in this step, as a result of performing optimization by changing only the number of nodes and hidden layers in the same manner as described above, a network consisting of two hidden layers and 20 nodes showed: Training Set AUC: 0.909 and Test Set AUC: 0.9453, and showed the best performance.

(3) Learning method or optimizer

A process of determining the weight value of the activation function for each node of the deep learning network is called "learning", and a hyperparameter that optimizes this learning quickly and stably is an optimizer. There are optimizers such as gradient descent and momentum, and an optimizer called ADADELTA provided in the H2o deep learning package was applied to this study. The ADADELTA optimizer has two parameters, Rho and Epsilon, Rho is a parameter which determines the rate of learning, and Epsilon is a parameter which determines the rate of decrease in learning. A test was performed by setting Rho and Epsilon in a range of 0.9 to 0.999 and 1E-4 to 1E-10, respectively. As a result of optimization, when Rho is 0.990 and Epsilon is 1E-8, Training Set AUC: 0.947 and Test Set AUC: 0.9252 are shown, showing the best performance.

(4) Epoch

Epoch refers to the number of times in which the training set data passes through the entire deep learning network once, that is, shows an effect that the data of the training set learns the entire deep learning network once. The degree of learning differs depending on the number of times, and the number of times of learning that represents the best performance was determined by learning in the cases of 10, 50, 100, 200, 300, 500, and 1000. In addition, when the number of times of learning exceeds a predetermined level, a phenomenon of saturation or decrease occurs without any further increase in performance, and when an excessively high number of times of learning is used, it takes a lot of time to learn, so that it is important to determine the number of times of learning for a suitable training set. As a result of epoch optimization, at 400 epochs, Training Set AUC: 0.909 and Test Set AUC: 0.9453 are shown, showing the best performance.

(5) L1 and L2 normalization

L1 and L2 normalization is intended to prevent overfitting during training of training sets in deep learning, and needs to be specified as appropriate values according to the data characteristics of the training set used. L1 normalization causes a model to ignore the corresponding variables by setting weighted values corresponding to unnecessary variables to 0, and L2 normalization makes a weighted value of an outlier with a very large or small value close to 0. That is, when the influence of other important variables is lost by concentrating a weighted value on one specific variable and dependence is made only on a specific variable with a high weighted value, an ability of a model to be generalized deteriorates, so an effort should be made such that the weighted value is not concentrated on a specific variable through L1 and L2 normalization.
Both L1 and L2 were tested for the commonly used three values 0, 1E-4, and 1E-5, and when L1 is 0 and L2 is E-5, Training Set AUC: 0.9301 and Test Set AUC: 0.9472 are shown, showing the best performance. As a result, L1 normalization was not applied, and only L2 normalization was applied.

(6) Dropout

Dropout is also a method devised to prevent the overfitting of learning in deep learning, and is a method of excluding some of the nodes arbitrarily selected in the repeated learning process from learning. Since Dropout has an effect of averaging many models, it is possible to reduce the time of learning, improve network performance and set a proportion of nodes to be eliminated. H2o may set the dropout ratio of the input layer and the hidden layer, respectively, and optimization was performed while increasing the ratio of by 0.05 in a range of 0 to 5. As a result of optimization, when the input layer is 0 and the hidden layer is 0.5, Training Set AUC: 0.9301, Test Set AUC: 0.9472 are shown, showing the best performance.

(7) Number of samples per one learning (Training samples per iteration)

The number of samples per one learning is a hyperparameter regarding how many samples are used as inputs when passing through the entire deep learning network once. A test was performed using a total of three values of 0, -1, and -2, and -2 showed the best performance.

(8) Max w2

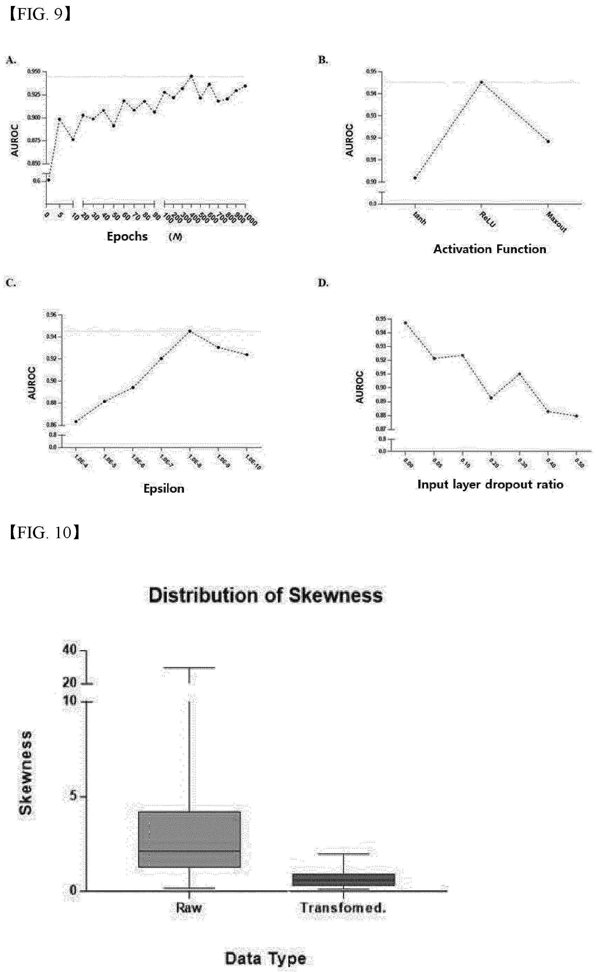

Max w2 serves to prevent the weighted value from becoming too large by setting the maximum upper limit of the weighted value, was tested from 0 to 10, and showed the best performance at 10. Optimization was performed on a total of 9 hyperparameters, and finally, the hyperparameters were completely optimized by setting the activation function to Relu, the numbers of nodes and hidden layers to 20 and 20, the optimizer to ADADELTA, the epoch to 400, L1 to 0 and L2 to 1E-5, the sample number value during one learning to -2, and finally Max w2 to 10, and the results are shown in FIG. 9.

### 3-2. Construction of biomarker panel using SVM method

### 3-2-1. Data preprocessing

As a first preprocessing process, in order to secure the reproducibility of MRM-MS analysis among the 68 peptides quantitatively analyzed by MRM-MS, a task of selecting only markers with a peak intensity of 1000 or higher was performed.

It was confirmed that a total of 11 peptides were measured with a peak intensity of 1000 or less by confirming the measurement intensity of each proteomics marker for each sample from MRM-MS Raw analysis data by Skyline software, and the corresponding marker was excluded from a proteomics marker candidate group from which a multi-marker panel was developed. Finally, a multi-marker panel was developed through 57 peptides. As a second preprocessing process, a data transformation task that matches the expression distribution of each proteomics marker candidate to the quantitative analysis data of large-scale blood samples obtained from multiple institutions. In order to distribute the expression distribution of proteomics marker candidates in a form close to the normal distribution, a total of five types of data transformation methods were applied: → Raw, Log (x+1), Log (x+10-10), Square, Square root. After the data was transformed with the 5 types of transformation methods, the expression distribution of proteomics marker candidates was confirmed by calculating the skewness value for each marker. Since the candidate proteomics markers may be expressed differently in pancreatic cancer and normal and pancreatic benign disease, the skewness values were calculated for the pancreatic cancer group and the control with normal and pancreatic benign disease, respectively. When a skewness value was calculated after data transformation was made for a total of 58 marker candidates obtained by combining 57 peptides and CA 19-9, it was confirmed that the marker candidates had a skewness from a minimum of 0.18 to a maximum of 28.89. After the skewness of each of the pancreatic cancer group and the control was obtained, data transformation was performed by finally selecting a data transformation method in which the sum of the absolute values of the skewness of each group was the smallest. As a final selection result of data transformation, Log (x+1) was 22, Log (x+10-10) was 11, Square root was 20, and Raw was 5. After the data transformation was performed, the skewness value of each marker was a minimum of 0.1 to a maximum of 1.9, confirming that all 58 markers were closer to the normal distribution form than before transformation.

[Table 6]

| Skewness | Raw | Transformed |
|---|---|---|
| Mean | 3.441 | 0.726 |
| Median | 2.122 | 0.588 |
| Min | 0.182 | 0.124 |
| Max | 29.892 | 1.964 |

### 3-2-2. Development of biomarker panel

The configuration of samples used to develop a multi-marker panel based on SVM is shown in Table 7. A multi-marker panel for diagnosing pancreatic cancer was developed using the data completely subjected to data preprocessing. A training set and a test set for developing a multi-marker panel were constructed using a total of 688 samples except for the samples from the Asan Medical Center institution. The training set consisted of 551 samples (261 cases of the patient group and 290 cases of the control) and the test set consisted of 137 samples (65 cases of the patient group and 72 cases of the control). The sample from the Asan Medical Center institution consisted of a single institutional independent validation cohort, and consisted of 75 cases of the patient group and 47 cases of the control. Finally, a multi-marker panel was developed and verified with one training set and two validation sets.

[Table 7]

| Sample | Institution | Number of samples | | | |
|---|---|---|---|---|---|
| | | Total | Training set | Test set | Independent validation set |
| Pancreatic cancer | Total | 401 | 261 | 65 | 75 |
| | Asan Medical Center | 75 | | | 75 |
| | National Cancer Center | 128 | 102 | 26 | |
| | Samsung Medical Cencter | 101 | 84 | 17 | |
| | Seoul National University Hospital | 50 | 38 | 12 | |
| | Yonsei Severance Hospital | 47 | 37 | 10 | |
| Pancreatic benign disease | Total | 109 | 47 | 15 | 47 |
| | Asan Medical Center | 47 | | | 47 |
| | Samsung Medical Cencter | 30 | 21 | 9 | |
| | Seoul National University Hospital | 5 | 3 | 2 | |
| | Yonsei Severance Hospital | 27 | 23 | 4 | |
| Normal | Seoul National University Hospital | 300 | 243 | 57 | |
| Total | | 810 | 551 | 137 | 122 |

The development and learning of a model were performed using a support vector machine (hereinafter, referred to as SVM) algorithm (R software version 3.6.0), which is one type of machine learning for the development of a multi-marker panel. Model learning and marker selection were performed using 10-fold cross validation to select a marker to be trained in the model from 57 proteomics marker candidates. As a result of developing a model, a multi-marker panel consisting of 12 SVM-based proteins was developed, and the combinations of proteins belonging to the multi-marker panel are as follows: LRG1, PPAP, C5, KLKB1, CLU, IGFBP2, THBS1, IFRD1, SERPINA5, TTHY, ICAM1, and VIM, and is also described in Table 8.

Furthermore, SVM parameters, cost and gamma, were also optimized when a model was constructed. 10-fold cross validation was performed, gamma was tuned in a range of 0 to 2, cost was tuned in a range of 1 to 100, and as a result of optimization, Kernel: Gaussian radial basis function (RBF), and gamma and cost were 0.15 and 2, respectively.

[Table 8]

| N. | Gene Symbol | Peptide Sequence |
|---|---|---|
| 1 | TTHY | AADDTWEPFASGK |
| 2 | CLU | ASSIIDELFQDR |
| 3 | LRG1 | DLLLPQPDLR |
| 4 | KLKB1 | DSVTGTLPK |
| 5 | IPSP | GFQQLLQELNQPR |
| 6 | IGFBP2 | LIQGAPTIR |
| 7 | C5 | NADYSYSVWK |
| 8 | IFRD1 | GLIDLTLDK |
| 9 | PPBP | NIQSLEVIGK |
| 10 | VIM | ILLAELEQLK |
| 11 | ICAM1 | LLGIETPLPK |
| 12 | THBS1 | TIVTTLQDSIR |

### 3-2-3. Performance of SVM biomarker panel

The diagnostic performance results of the 12 SVM multi-marker panels are as follows:

- Training set: an AUC of 0.993, a sensitivity of 94.6%, a specificity of 98.3%, and an accuracy of 96.6%
- Test set: an AUC of 0.961, a sensitivity of 92.3%, a specificity of 93.1%, and an accuracy of 92.7%

The performance results of CA19-9 alone in the training set and test set are as follows:

- Training set: an AUC of 0.872, a sensitivity of 71.7%, a specificity of 98.9%, and an accuracy of 86%
- Test set: an AUC of 0.828, a sensitivity of 58.5%, a specificity of 97.2%, and an accuracy of 78.8%

As a result of statistical comparison of the increase in AUC of the multi-marker panel and CA 19-9 alone, it was confirmed that both the training set and the test showed a significant difference of P < 0.01 or < 0.001 (DeLongs Test) and had an increase in diagnostic ability to distinguish pancreatic cancer. After a model was created by combining CA19-9 with a multi-marker panel developed to investigate the characteristics as a complementary marker to CA19-9, which is currently used as a blood marker for diagnosing pancreatic cancer, a validation task was performed in the same manner in the training set and the test set.

The performance results of a model obtained by combining the SVM-based multi-marker panel and CA19-9 are as follows:

- Training set: an AUC of 0.996, a sensitivity of 96.9%, a specificity of 99.6%, and an accuracy of 98.4%
- Test set: an AUC of 0.983, a sensitivity of 86.2%, a specificity of 97.2%, and an accuracy of 92%

As a result of statistical comparison of the increase in AUC of the multi-marker panel and CA 19-9 combined model, it was confirmed that both the training set and the test set showed a significant difference of *P < 0.001* (DeLongs Test) and had an increase in diagnostic ability to distinguish pancreatic cancer.

**Diagnostic performance validation of multi-marker panel in independent validation set**

Diagnostic performance was evaluated in a single institutional independent validation set to increase the generality and reliability of the multi-marker panel and secure clinical utility applicable to different cohorts. The independent validation set consisted of samples collected from Asan Medical Center, and consisted of a total of 122 cases including 75 cases of the patient group and 47 cases of the control.

The results of the multi-marker panel performance evaluation in the independent validation set are as follows:

- an AUC of 0.945, a sensitivity of 94.7%, a specificity of 80.9%, and an accuracy of 89.3%.

The performance evaluation results of CA 19-9 alone in the independent validation set are as follows.

- an AUC of 0.771, a sensitivity of 61.3%, a specificity of 95.7%, and an accuracy of 75%.

As a result of statistical comparison of the increase in AUC of the multi-marker panel and CA 19-9 alone, it was confirmed that the independent validation set showed a significant difference of P < 0.001 (DeLongs Test) and had an increase in diagnostic ability to distinguish pancreatic cancer.

The results obtained when a combined model of the multi-marker panel and CA 19-9 is evaluated in the independent validation set are as follows.

- an AUC of 0.96, a sensitivity of 96%, a specificity of 85.1%, and an accuracy of 91.8%.

As a result of statistical comparison of the AUC difference with CA 19-9 alone, it was confirmed that the independent validation set showed a significant difference of P < 0.001 (DeLongs Test) and had an increase in diagnostic ability to distinguish pancreatic cancer.

**Validation of diagnostic performance of multi-marker panel in subgroup**

Study results have shown that CA19-9, an existing marker for diagnosing pancreatic cancer, increases not only in pancreatic cancer but also in other malignant tumors such as liver cancer, colorectal cancer, and lung cancer. Therefore, such non-specific expression of CA 19-9 is shown as a decrease in sensitivity during the diagnosis of pancreatic cancer. The performance of the multi-marker panel was evaluated by constructing a subgroup in order to secure the specificity of the multi-marker panel and investigate properties complementary to the limitations of CA 19-9. The subgroup constituted a total of two additional cohorts.

- Additional Cohort 1: 50 cases of pancreatic cancer and 149 cases of other carcinomas
- Additional Cohort 2: 50 cases of pancreatic cancer, 149 cases of other carcinomas, and 57 cases of normal persons
- Patient group: pancreatic cancer, control: other carcinomas or other carcinomas + normal

The evaluation results of the multi-marker panel in two subgroups are as follows.

- Additional Cohort 1: an AUC of 0.936, a sensitivity of 94%, a specificity of 62.4%, and an accuracy of 70.3%
- Additional Cohort 2: an AUC of 0.951, a sensitivity of 94%, a specificity of 71.8%, and an accuracy of 77.1%

The evaluation results of a model obtained by combining the multi-marker panel and CA 19-9 in two subgroups are as follows.

- Additional Cohort 1: an AUC of 0.966, a sensitivity of 96%, a specificity of 87.9%, and an accuracy of 90%
- Additional Cohort 2: an AUC of 0.972, a sensitivity of 96%, a specificity of 90.29%, and an accuracy of 92.3%

The evaluation results of CA 19-9 alone in two subgroups are as follows.

- Additional Cohort 1: an AUC of 0.886, a sensitivity of 76%, a specificity of 92.6%, and an accuracy of 88.4%
- Additional Cohort 2: an AUC of 0.894, a sensitivity of 76%, a specificity of 93.7%, and an accuracy of 91.1%

As a result of the evaluation, both the multi-marker panel and the combined model showed a high diagnostic ability to distinguish pancreatic cancer with an AUC of 0.93 or more. When a statistical comparative analysis of the AUC values of CA 19-9 alone and the multi-marker panel was performed, it was confirmed that the multi-marker panel alone did not show a significant difference, and the model obtained by combining the panel with CA 19-9 had a significant increase in AUC of P <0.05 (DeLongs Test).

**3-3. Construction of biomarker panel using logistic regression method**

A multi-marker panel was additionally constructed using a logistic regression method. MRM-MS quantitative analysis data of a total of 810 cases was used in the samples used, and consisted of 649 cases of a training set (disease group: 321 cases, control: 328 cases) and 161 cases of a test set (disease group: 80 cases, control: 81 cases). The pancreatic cancer group was set as a disease group, and the normal and pancreatic benign disease were combined and set as a control. When a multi-marker panel was developed using logistic regression, the marker selection process used a stepwise selection method, and a marker was selected and a model was developed using the selection criteria of p-value < 0.05 or less and an Akaike information criterion (AIC). As a result of developing a model by logistic regression, a total of 15 models were developed, and all the 15 models showed an AUC > 0.85 or more, and showed an excellent diagnostic ability to distinguish pancreatic cancer. The respective markers that constituted the 15 models are summarized in Tables 9-1 and 9-2.

[Table 9-1]

| N. | N. of Markers | Markers |
|---|---|---|
| 1 | 14 | CLU_ASSIIDELFQDR, C5_NADYSYSVWK, KLKB1_DSVTGTLPK, PPBP_NIQSLEVIGK, IFRD1_GLIDLTLDK, IGFBP2_LIQGAPTIR, ICAM1_LLGIETPLPK, C4BPA_LSLEIEQLELQR, PTPRJ_DTEVLLVGLEPGTR, ECM1_ELLALIQLER, VIM_ILLAELEQLK, C4BPB_ALLAFQESK, IPS?_GFQQLLQELNQPR, TTHY_AADDTWEPFASGK |
| 2 | 17 | CLU_ASSIIDELFQDR, C5_NADYSYSVWK, KLKB1_DSVTGTLPK, PPBP_NIQSLEVIGK, ICAM1_LLGIETPLPK, IFRD1_GLIDLTLDK, IGFBP2_LIQGAPTIR, VIM_ILLAELEQLK, PTPRJ_DTEVLLVGLEPGTR, ECM1_ELLALIQLER, GSTP1_ASCLYGQLPK, C4BPA_LSLEIEQLELQR, C4BPB_ALLAFQESK, IPSP_GFQQLLQELNQPR, TTHY_AADDTWEPFASGK, CPN2_LSNNALSGLPQGVFGK, APOH_VCPFAGILENGAVR |
| 3 | 16 | THBS1_TIVTTLQDSIR, IGFBP3_ALAQCAPPPAVCAELVR, C5_NADYSYSVWK, CLU_TLLSNLEEAK, IGFBP2_LIQGAPTIR, ECM1_ELLALIQLER, ICAM1_LLGIETPLPK, CLU_ASSIIDELFQDR, SERPINC1_IEDGFSLK, CFI_VFSLQWGEVK, ADIPO_GDIGETGVPGAEGPR, PTPRJ_DTEVLLVGLEPGTR, KLK31_DSVTGTLPK, C6_TLNICEVGTIR, PPBP_NIQSLEVIGK, C4BPA_LSLEIEQLELQR |
| 4 | 23 | TTHY_AADDTWEPFASGK, ITIH4_AGFSWIEVTFK, C4BPB_ALLAFQESK, CLU_ASSIIDELFQDR, SEPP1_CINQLLCK, KLKB1_DSVTGTLPK, C1R_DYFIATCK, PTPRJ_DTEVLLVGLEPGTR, ECM1_ELLALIQLER, ADIPO_GDIGETGVPGAEGPR, IPSP_GFQQLLQELNQPR, VIM_ILLAELEQLK, ICAM1_LLGIETPLPK, GFBP2_LIQGAPTIR, C4BPA_LSLEIEQLELQR, C5_NADYSYSVWK, PPBP_NIQSLEVIGK, THBS1_TIVTTLQDSIR, C1S_TNFDNDIALVR, C6_TLNICEVGTIR, SERPINC1_VAEGTQVLELPFK, APOH_VCPFAGILENGAVR, IGFBP3_YGQPLPGYTTK |
| 5 | 12 | THBS1_TIVTTLQDSIR, IGFBP2_LIQGAPTIR, C5_NADYSYSVWK, CLU_TLLSNLEEAK, ECM1_ELLALIQLER, ICAM1_LLGIETPLPK, TTHY_AADDTWEPFASGK, C4BPA_LSLEIEQLELQR, IFRD1_GLIDLTLDK, KLKB1_DSVTGTLPK, C1R_DYFIATCK, C4BPB_ALLAFQESK |
| 6 | 17 | THBS1_TIVTTLQDSIR, IGFBP2_LIQGAPTIR, IGFBP3_ALAQCAPPPAVCAELVR, C5_NADYSYSVWK, CLU_TLLSNLEEAK, ECM1_ELLALIQLER, ICAM1_LLGIETPLPK, TTHY_AADDTWEPFASGK, C4BPA_LSLEIEQLELQR, C7_VLFYVDSEK, CLU_ASSIIDELFQDR, SERPINC1_IEDGFSLK, C4BPA_TWYPEVPK, PPBP_NIQSLEVIGK, IPSP_GFQQLLQELNQPR, VIM_ILLAELEQLK, LDHB_GLTSVINQK |
| 7 | 21 | TTHY_AADDTWEPFASGK, C4BPB_ALLAFQESK, CLU_ASSIIDELFQDR, C1R_DYFIATCK, ECM1_ELLALIQLER, IPSP_GFQQLLQELNQPR, LDHB_GLTSVINQK, COL4A2_GLPGEVLGAQPGPR, SERPINC1_IEDGFSLK, VIM_ILLAELEQLK, ICAM1_LLGIETPLPK, IGFBP2_LIQGAPTIR, C4BPA_LSLEIEQLELQR, C5_NADYSYSVWK, PPBP_NIQSLEVIGK, CLU_TLLSNLEEAK, C1S_TNFDNDIALVR, LRG1_VAAGAFQGLR, APOH_VCPFAGILENGAVR, C7_VLFYVDSEK, IGFBP3_YGQPLPGYTTK |

[Table 9-2]

| N. | N. of Markers | Markers |
|---|---|---|
| 8 | 14 | THBS1_TIVTTLQDSIR, HSPG2_SPAYTLVWTR, TTHY_AADDTWEPFASGK, IFRD1_GLIDLTLDK, IGFBP2_LIQGAPTIR, ECM1_ELLALIQLER, ICAM1_LLGIETPLPK, SFTPB_FLEQECNVLPLK, SPARC_NVLVTLYER, C4BPA_LSLEIEQLELQR, KLKB1_DSVTGTLPK, IPSP_GFQQLLQELNQPR, C5_NADYSYSVWK, COL4A2_GLPGEVLGAQPGPR |
| 9 | 16 | TTHY_AADDTWEPFASGK, KLKB1_DSVTGTLPK, PTPRJ_DTEVLLVGLEPGTR, ECM1_ELLALIQLER, SFTPB_FLEQECNVLPLK, IPSP_GFQQLLQELNQPR, COL4A2_GLPGEVLGAQPGPR, ITIH2_IQPSGGTNINEALLR, ICAM1_LLGIETPLPK, IGFBP2_LIQGAPTIR, CPN2_LSNNALSGLPQGVFGK, C4BPA_LSLEIEQLELQR, C5_NADYSYSVWK, SPARC_NVLVTLYER, HSPG2_SPAYTLVWTR, THBS1_TIVTTLQDSIR |
| 10 | 16 | PPBP_ICLDPDAPR, HRG_DGYLFQLLR, PKM2_CDENILWLDYK, LRG1_VAAGAFQGLR, AGT_DPTFIPAPIQAK, ICAM1_VTLNGVPAQPLGPR, PDCD4_GDSVSDSGSDALR, C7_VLFYVDSEK, C5_GGSASTWLTAFALR, ITIH4_LALDNGGLAR, CTSD_VSTLPAITLK, IGFBP3_YGQPLPGYTTK, C4BPA_TWYPEVPK, LDHB_GLTSVINQK, APOC1_EFGNTLEDK, LDHB_IVVVTAGVR |
| 11 | 20 | PKM2_CDENILWLDYK, HRG_DGYLFQLLR, AGT_DPTFIPAPIQAK, APOC1_EFGNTLEDK, PDCD4_GDSVSDSGSDALR, THBS1_GFLLLASLR, C5_GGSASTWLTAFALR, LDHB_GLTSVINQK, PPBP_ICLDPDAPR, LDHB_IVVVTAGVR, ITIH4_LALDNGGLAR, CFH_TGESVEFVCK, C4BPA_TWYPEVPK, C1S_TNFDNDIALVR, LRG1_VAAGAFQGLR, C7_VLFYVDSEK, ICAM1_VTLNGVPAQPLGPR, IGFBP3_YGQPLPGYTTK, CTSD_VSTLPAITLK, SOD3_VTGVVLFR |
| 12 | 23 | PKM2_CDENILWLDYK, TXN_CMPTFQFFK, HRG_DGYLFQLLR, AGT_DPTFIPAPIQAK, APOC1_EFGNTLEDK, APOC1_EWFSETFQK, CFH_GEWVALNPLR, PDCD4_GDSVSDSGSDALR, THBS1_GFLLLASLR, C5_GGSASTWLTAFALR, LDHB_GLTSVINQK, PPBP_ICLDPDAPR, LDHB_IVVVTAGVR, ITIH4_LALDNGGLAR, FSTL1_LSFQEFLK, CFH_TGESVEFVCK, C4BPA_TWYPEVPK, C1S_TNFDNDIALVR, LRG1_VAAGAFQGLR, C7_VLFYVDSEKI, CAM1_VTLNGVPAQPLGPR, IGFBP3_YGQPLPGYTTK, CTSD_VSTLPAITLK |
| 13 | 14 | PPBP_ICLDPDAPR, HRG_DGYLFQLLR, LRG1_VAAGAFQGLR, PDCD4_GDSVSDSGSDALR, AGT_DPTFIPAPIQAK, MBL2_FQASVATPR, PKM2_CDENILWLDYK, ICAM1_VTLNGVPAQPLGPR, THBS1_GFLLLASLR, C5_GGSASTWLTAFALR, ITIH4_LALDNGGLAR, C7_VLFYVDSEK, APOC1_EFGNTLEDK, CFH_TGESVEFVCK |
| 14 | 12 | LRG1_DLLLPQPDLR, SEPP1_CINQLLCK, IGFBP3_ALAQCAPPPAVCAELVR, CLU_TLLSNLEEAK, SERPINC1_IEDGFSLK, SERPINC1_VAEGTQVLELPFK, C1R_DYFIATCK, P4HB_ALAPEYAK, CDH11_VLDVNDNAPK, FCGBP_FAVLQENVAWGNGR, CPN2_LSNNALSGLPQGVFGK, BTD_ILSGDPYCEK |
| 15 | 14 | P4HB_ALAPEYAK, CORO1C_CDLISIPK, FCGBP_FAVLQENVAWGNGR, MMSA_QGIQFYTQLK, SERPINC1_VAEGTQVLELPFK, SEPP1_CINQLLCK, LRG1_DLLLPQPDLR, C1R_DYFIATCK, BTD_ILSGDPYCEK, CPN2_LSNNALSGLPQGVFGK, IGFBP3_ALAQCAPPPAVCAELVR, CLU_TLLSNLEEAK, SERPINC1_IEDGFSLK, CAP1_EPAVLELEGK |

The performance of each panel of the 15 logistic regression-based multi-marker panels is as follows. Each model below shows a combination of markers.

- Model 1: Training set: an AUC of 0.977, a sensitivity of 90.42%, and a specificity of 95.86%, Test set: an AUC of 0.953, a sensitivity of 84.62%, and a specificity of 94.44%

- Model 2: Training set: an AUC of 0.979, a sensitivity of 90.4%, and a specificity of 96.2%, Test set: an AUC of 0.952, a sensitivity of 81.5%, and a specificity of 94.4%

- Model 3: Training set: an AUC of 0.981, a sensitivity of 94.25%, and a specificity of 93.44%, Test set: an AUC of 0.954, a sensitivity of 87.69%, and a specificity of 88.89%

- Model 4: Training set: an AUC of 0.986, a sensitivity of 93.86%, and a specificity of 96.2%, Test set: an AUC of 0.956, a sensitivity of 84.62%, and a specificity of 91.67%
- Model 5: Training set: an AUC of 0.976, a sensitivity of 93.1%, and a specificity of 92.4%, Test set: an AUC of 0.964, a sensitivity of 95.38%, and a specificity of 88.89%
- Model 6: Training set: an AUC of 0.978, a sensitivity of 93.07%, and a specificity of 94.15%, Test set: an AUC of 0.968, a sensitivity of 90.91%, and a specificity of 94.37%
- Model 7: Training set: an AUC of 0.982, a sensitivity of 94.23%, and a specificity of 94.16%, Test set: an AUC of 0.968, a sensitivity of 90.91%, and a specificity of 82.96%
- Model 8: Training set: an AUC of 0.971, a sensitivity of 90.65%, and a specificity of 93.9%, Test set: an AUC of 0.943, a sensitivity of 90%, and a specificity of 91.36%

- Model 9: Training set: an AUC of 0.974, a sensitivity of 92.21%, and a specificity of 92.07%, Test set: an AUC of 0.946, a sensitivity of 93.75%, and a specificity of 85.19%
- Model 10: Training set: an AUC of 0.919, a sensitivity of 83.17%, and a specificity of 88.41%, Test set: an AUC of 0.901, a sensitivity of 81.25%, and a specificity of 77.78%
- Model 11: Training set: an AUC of 0.92, a sensitivity of 82.55%, and a specificity of 87.5%, Test set: an AUC of 0.908, a sensitivity of 83.75%, and a specificity of 88.89%
- Model 12: Training set: an AUC of 0.918, a sensitivity of 86.91%, and a specificity of 84.76%, Test set: an AUC of 0.922, a sensitivity of 82.5%, and a specificity of 90.12%
- Model 13: Training set: an AUC of 0.921, a sensitivity of 85.67%, and a specificity of 88.41%, Test set: an AUC of 0.875, a sensitivity of 83.75%, and a specificity of 80.25%
- Model 14: Training set: an AUC of 0.898, a sensitivity of 75.07%, and a specificity of 91.16%, Test set: an AUC of 0.855, a sensitivity of 71.25%, and a specificity of 83.95%
- Model 15: Training set: an AUC of 0.899, a sensitivity of 83.49%, and a specificity of 82.93%, Test set: an AUC of 0.858, a sensitivity of 76.25%, and a specificity of 76.54%

**Example 4. Analytical information of 19 markers for diagnosing pancreatic cancer by mass spectrometer and use thereof**

In Example 3, the markers in which duplicates are removed from the markers selected from Machine Learning (Deep Learning, Support vector machine, Logistic regression) totaled 68 peptides (see Tables 10-1 to 10-4).

The information used during each transition analysis according to the specific collision energy using an Agilent 6400 Series Triple quadrupole mass spectrometer for the finally selected 19 peptides is as follows. When analysis was performed using a mass spectrometer, the collision energy was used for the analysis of the transition intensity analyzed through the increase and decrease by $\pm$ 2V by 5 points from a default value predetermined by the 6400 Series. When an analysis is performed using the Agilent 6400 Series Triple quadrupole, a calibration curve analysis was additionally performed in order to select a Top-3 transition which has reproducibility, exhibits linearity, and can be stably analyzed. Analysis was performed with 18 points during the calibration curve analysis, and the amount of SIS-peptide spiked at each point is as follows: 2000, 1000, 500, 250, 125, 62.5, 31.25, 15.63, 7.81, 3.91, 1.95, 0.98, 0.49, 0.24, 0.12, 0.06, 0.03, 0.02 fmol. For the selection criteria for the Top-3 transition of 68 peptides, the Top-3 transition was selected in consideration of the fact that as the concentration goes to the low point, the trend of decreasing intensity is sharp, the dynamic range is wide, interference is low at the low concentration, the ion is long, and the y-ion is prioritized

Information on Top-3 transition and collision energy for 68 peptides that are reproducible and can be stably analyzed is as follows: AADDTWEPFASGK - y9, y8, y7 (22.6); AGFSWIEVTFK - y8, y6, b9 (20.9); ASSIIDELFQDR - y8, y7, y6 (22.6); CINQLLCK - y7, y6, b5 (17.3); DLLLPQPDLR - y8, y7, y6 (19.3); DSVTGTLPK - y7, y6, y5 (15.2); DYFIATCK - y6, y5, b4 (16.8); GFQQLLQELNQPR - y7, y6, y5 (14.1); ILSGDPYCEK - y8, y7, y5(19.3); LIQGAPTIR - y8, y7, y6 (16); LSSGLVTAALYGR - y9, y8, y7 (21.3); NADYSYSVWK - y8, y7, y6 (20.1); NNLELSTPLK - y8, y7, y6 (18.5); VFSLQWGEVK - y9, y8, y6(19.5); GLIDLTLDK - b2, y2, y7 (17.4); NIQSLEVIGK - b2, y8, y2 (19.4); ILLAELEQLK - b2, y8, y6 (20.7); LLGIETPLPK - y8, y6, b3 (19.1); TIVTTLQDSIR - b2, y8, y9 (22.0); ALAPEYAK - y6, y5, y3 (15.2); ALAQCAPPPAVCAELVR - y6, y5, b6 (15.5); ALLAFQESK - y7, y6, b2 (17.7); ASCLYGQLPK - y5, y2, b3 (20.1); CDENILWLDYK - y5, y4, y2 (26); CDLISIPK - y4, y2, b2 (16.7); CMPTFQFFK - y7, y5, y2 (21.3); DGYLFQLLR - y6, y5, b3 (19.9); DPTFIPAPIQAK - y7, b4, b5 (23); DTEVLLVGLEPGTR - y7, y4, b6 (26.6); EFGNTLEDK - y7, y4, y2 (18.6); ELLALIQLER - y7, y6, y5 (21.2); EPAVLELEGK - y6, y4, y2 (19.1); EWFSETFQK - y7, y6, y4 (21.2); FAVLQENVAWGNGR - y6, y5, y4 (27.7); FLEQECNVLPLK - y7, y4, y3 (26.4); FQASVATPR - y7, y6, b2 (17.2); GDIGETGVPGAEGPR - y9, y7, b8 (25); GDSVSDSGSDALR - y9, y7, y6 (22.4); GEWVALNPLR - y6, y5, y4 (20.4); GFLLLASLR - y5, y4, b4 (17.4); GGSASTWLTAFALR - y8, y7, y6 (25.5); GLPGEVLGAQPGPR - y6, y5, y4 (23.9); GLTSVINQK - y7, y4, b2 (16.9); ICLDPDAPR - y7, y6, b2 (18.6); IEDGFSLK - y7, y6, b2 (16); IQPSGGTNINEALLR - y6, y4, b2 (12.3); IVVVTAGVR - y7, y6, b2 (16.1); LALDNGGLAR - y8, y6, b2 (17.6); LSFQEFLK - y7, y6, b2 (17.8); LSLEIEQLELQR - y7, y5, y4 (26.1); LSNNALSGLPQGVFGK - y10, y7, y3 (28.4); NVLVTLYER - y7, b2, b3 (19.5); QGIQFYTQLK - y5, y4, y2 (21.7); SPAYTLVWTR - y6, y5, y3 (21.1); TGESVEFVCK - y5, y2, b3 (20.4); TLLSNLEEAK - y8, y7, b2 (19.7); TLNICEVGTIR - y7, y6, y4 (22.6); TNFDNDIALVR - y8, y4, b2 (22.6); TWYPEVPK - y5, y2, b2 (18); VAAGAFQGLR - y8, y7, b2 (17.4); VAEGTQVLELPFK - y6, y4, y3 (25.3); VCPFAGILENGAVR - y12, y9, b2 (26.6); VLDVNDNAPK - y8, y6, b2 (19.1); VLFYVDSEK - y7, y6, b2 (19.4); VSTLPAITLK - y7, b2, b3 (18.4); VTGVVLFR - y6, y4, b2 (15.7); VTLNGVPAQPLGPR - y8, y5, b6 (25.1); YGQPLPGYTTK - y8, y3, b3 (21.7);

Information on Top-3 transition and collision energy of markers for diagnosing pancreatic cancer is shown in Tables 10-1 to 10-4.

[Table 10-1]

| N. | Gene Symbol | Peptide Sequence | Precursor Charge | Product Charge | Fragment Ion | Precursor (m/z) | Product (m/z) | Collision Energy(V) |
|---|---|---|---|---|---|---|---|---|
| 1 | TTHY | AADDTWEPFASGK | 2 | 1 | y9 | 697.8 | 1022.5 | 22.6 |
| | | | 2 | 1 | y8 | 697.8 | 921.4 | 22.6 |
| | | | 2 | 1 | y7 | 697.8 | 735.4 | 22.6 |
| 2 | ITIH4 | AGFSWIEVTFK | 2 | 1 | y8 | 642.8 | 1009.5 | 20.9 |
| | | | 2 | 1 | y6 | 642.8 | 736.4 | 20.9 |
| | | | 2 | 1 | b9 | 642.8 | 991.5 | 20.9 |
| 3 | CLU | ASSIIDELFQDR | 2 | 1 | y8 | 697.4 | 1035.5 | 22.6 |
| | | | 2 | 1 | y7 | 697.4 | 922.4 | 22.6 |
| | | | 2 | 1 | y6 | 697.4 | 807.4 | 22.6 |
| 4 | SEPP1 | CINQLLCK | 2 | 1 | y7 | 524.8 | 888.5 | 17.3 |
| | | | 2 | 1 | y6 | 524.8 | 775.4 | 17.3 |
| | | | 2 | 1 | b5 | 524.8 | 629.3 | 17.3 |
| 5 | LRG1 | DLLLPQPDLR | 2 | 1 | y8 | 590.3 | 951.6 | 19.3 |
| | | | 2 | 1 | y7 | 590.3 | 838.5 | 19.3 |
| | | | 2 | 1 | y6 | 590.3 | 725.4 | 19.3 |
| 6 | KLKB1 | DSVTGTLPK | 2 | 1 | y7 | 459.3 | 715.4 | 15.2 |
| | | | 2 | 1 | y6 | 459.3 | 616.4 | 15.2 |
| | | | 2 | 1 | y5 | 459.3 | 515.3 | 15.2 |
| 7 | C1R | DYFIATCK | 2 | 1 | y6 | 509.2 | 739.4 | 16.8 |
| | | | 2 | 1 | y5 | 509.2 | 592.3 | 16.8 |
| | | | 2 | 1 | b4 | 509.2 | 539.3 | 16.8 |
| 8 | IPSP | GFQQLLQELNQPR | 3 | 1 | y7 | 524.3 | 884.5 | 14.1 |
| | | | 3 | 1 | y6 | 524.3 | 756.4 | 14.1 |
| | | | 3 | 1 | y5 | 524.3 | 627.4 | 14.1 |
| 9 | BTD | ILSGDPYCEK | 2 | 1 | y8 | 591.3 | 955.4 | 19.3 |
| | | | 2 | 1 | y7 | 591.3 | 868.4 | 19.3 |
| | | | 2 | 1 | y5 | 591.3 | 696.3 | 19.3 |
| 10 | IGFBP2 | LIQGAPTIR | 2 | 1 | y8 | 484.8 | 855.5 | 16 |
| | | | 2 | 1 | y7 | 484.8 | 742.4 | 16 |
| | | | 2 | 1 | y6 | 484.8 | 614.4 | 16 |
| 11 | BTD | LSSGLVTAALYGR | 2 | 1 | y9 | 654.4 | 963.6 | 21.3 |
| | | | 2 | 1 | y8 | 654.4 | 850.5 | 21.3 |
| | | | 2 | 1 | y7 | 654.4 | 751.4 | 21.3 |
| 12 | C5 | NADYSYSVWK | 2 | 1 | y8 | 616.8 | 1047.5 | 20.1 |
| | | | 2 | 1 | y7 | 616.8 | 932.5 | 20.1 |
| | | | 2 | 1 | y6 | 616.8 | 769.4 | 20.1 |
| 13 | PROS1 | NNLELSTPLK | 2 | 1 | y8 | 564.8 | 900.5 | 18.5 |
| | | | 2 | 1 | y7 | 564.8 | 787.5 | 18.5 |
| | | | 2 | 1 | y6 | 564.8 | 658.4 | 18.5 |
| 14 | CFI | VFSLQWGEVK | 2 | 1 | y9 | 596.8 | 1093.6 | 19.5 |
| | | | 2 | 1 | y8 | 596.8 | 946.5 | 19.5 |
| | | | 2 | 1 | y6 | 596.8 | 746.4 | 19.5 |
| 15 | IFRD1 | GLIDLTLDK | 2 | 1 | b2 | 494.3 | 171.1 | 17.4 |
| | | | 2 | 1 | y2 | 494.3 | 262.1 | 17.4 |
| | | | 2 | 1 | y7 | 494.3 | 817.5 | 17.4 |
| 16 | PPBP | NIQSLEVIGK | 2 | 1 | b2 | 550.8 | 228.1 | 19.4 |
| | | | 2 | 1 | y8 | 550.8 | 873.5 | 19.4 |
| | | | 2 | 1 | y2 | 550.8 | 204.1 | 19.4 |
| 17 | VIM | ILLAELEQLK | 2 | 1 | b2 | 585.4 | 227.2 | 20.7 |
| | | | 2 | 1 | y8 | 585.4 | 943.5 | 20.7 |
| | | | 2 | 1 | y6 | 585.4 | 759.4 | 20.7 |

[Table 10-2]

| N. | Gene Symbol | Peptide Sequence | Precursor Charge | Product Charge | Fragment Ion | Precursor (m/z) | Product (m/z) | Collision Energy(V) |
|---|---|---|---|---|---|---|---|---|
| 18 | ICAM1 | LLGIETPLPK | 2 | 1 | y8 | 540.8 | 854.5 | 19.1 |
| | | | 2 | 1 | y6 | 540.8 | 684.4 | 19.1 |
| | | | 2 | 1 | b3 | 540.8 | 284.2 | 19.1 |
| 19 | THBS1 | TIVTTLQDSIR | 2 | 1 | b2 | 623.9 | 215.1 | 22.0 |
| | | | 2 | 1 | y8 | 623.9 | 933.5 | 22.0 |
| | | | 2 | 1 | y9 | 623.9 | 1032.6 | 22.0 |
| 20 | ADIPOQ | GDIGETGVPGAEGPR | 2 | 1 | y9 | 706.3 | 839.4 | 25 |
| | | | 2 | 1 | y7 | 706.3 | 683.3 | 25 |
| | | | 2 | 1 | b8 | 706.3 | 729.3 | 25 |
| 21 | AGT | DPTFIPAPIQAK | 2 | 1 | y7 | 649.4 | 724.4 | 23 |
| | | | 2 | 1 | b4 | 649.4 | 461.2 | 23 |
| | | | 2 | 1 | b5 | 649.4 | 574.3 | 23 |
| 22 | ALDH6A1 (MMSA) | QGIQFYTQLK | 2 | 1 | y5 | 613.3 | 652.4 | 21.7 |
| | | | 2 | 1 | y4 | 613.3 | 489.3 | 21.7 |
| | | | 2 | 1 | y2 | 613.3 | 260.2 | 21.7 |
| 23 | APOC1 | EFGNTLEDK | 2 | 1 | y7 | 526.7 | 776.4 | 18.6 |
| | | | 2 | 1 | y4 | 526.7 | 504.3 | 18.6 |
| | | | 2 | 1 | y2 | 526.7 | 262.1 | 18.6 |
| 24 | APOC1 | EWFSETFQK | 2 | 1 | y7 | 601.3 | 886.4 | 21.2 |
| | | | 2 | 1 | y6 | 601.3 | 739.4 | 21.2 |
| | | | 2 | 1 | y4 | 601.3 | 523.3 | 21.2 |
| 25 | APOH | VCPFAGILENGAVR | 2 | 1 | y12 | 751.9 | 1243.7 | 26.6 |
| | | | 2 | 1 | y9 | 751.9 | 928.5 | 26.6 |
| | | | 2 | 1 | b2 | 751.9 | 260.1 | 26.6 |
| 26 | C1S | TNFDNDIALVR | 2 | 1 | y8 | 639.3 | 915.5 | 22.6 |
| | | | 2 | 1 | y4 | 639.3 | 458.3 | 22.6 |
| | | | 2 | 1 | b2 | 639.3 | 216.1 | 22.6 |
| 27 | C4BPA | TWYPEVPK | 2 | 1 | y5 | 510.3 | 569.3 | 18 |
| | | | 2 | 1 | y2 | 510.3 | 244.2 | 18 |
| | | | 2 | 1 | b2 | 510.3 | 288.1 | 18 |
| 28 | C4BPA | LSLEIEQLELQR | 2 | 1 | y7 | 735.9 | 915.5 | 26.1 |
| | | | 2 | 1 | y5 | 735.9 | 658.4 | 26.1 |
| | | | 2 | 1 | y4 | 735.9 | 545.3 | 26.1 |
| 29 | C4BPB | ALLAFQESK | 2 | 1 | y7 | 503.8 | 822.4 | 17.7 |
| | | | 2 | 1 | y6 | 503.8 | 709.4 | 17.7 |
| | | | 2 | 1 | b2 | 503.8 | 185.1 | 17.7 |
| 30 | C5 | GGSASTWLTAFALR | 2 | 1 | y8 | 719.4 | 977.6 | 25.5 |
| | | | 2 | 1 | y7 | 719.4 | 791.5 | 25.5 |
| | | | 2 | 1 | y6 | 719.4 | 678.4 | 25.5 |
| 31 | C6 | TLNICEVGTIR | 2 | 1 | y7 | 638.3 | 834.4 | 22.6 |
| | | | 2 | 1 | y6 | 638.3 | 674.4 | 22.6 |
| | | | 2 | 1 | y4 | 638.3 | 446.3 | 22.6 |
| 32 | C7 | VLFYVDSEK | 2 | 1 | y7 | 550.3 | 887.4 | 19.4 |
| | | | 2 | 1 | y6 | 550.3 | 740.3 | 19.4 |
| | | | 2 | 1 | b2 | 550.3 | 213.2 | 19.4 |
| 33 | CAP1 | EPAVLELEGK | 2 | 1 | y6 | 542.8 | 688.4 | 19.1 |
| | | | 2 | 1 | y4 | 542.8 | 446.3 | 19.1 |
| | | | 2 | 1 | y2 | 542.8 | 204.1 | 19.1 |
| 34 | CDH11 | VLDVNDNAPK | 2 | 1 | y8 | 542.8 | 872.4 | 19.1 |
| | | | 2 | 1 | y6 | 542.8 | 658.3 | 19.1 |
| | | | 2 | 1 | b2 | 542.8 | 213.2 | 19.1 |
| 35 | CFH | GEWVALNPLR | 2 | 1 | y6 | 577.8 | 683.4 | 20.4 |
| | | | 2 | 1 | y5 | 577.8 | 612.4 | 20.4 |
| | | | 2 | 1 | y4 | 577.8 | 499.3 | 20.4 |

[Table 10-3]

| N. | Gene Symbol | Peptide Sequence | Precursor Charge | Product Charge | Fragment Ion | Precursor (m/z) | Product (m/z) | Collision Energy(V) |
|---|---|---|---|---|---|---|---|---|
| 36 | CFH | TGESVEFVCK | 2 | 1 | y5 | 578.3 | 682.3 | 20.4 |
| | | | 2 | 1 | y2 | 578.3 | 307.1 | 20.4 |
| | | | 2 | 1 | b3 | 578.3 | 288.1 | 20.4 |
| 37 | CLU | TLLSNLEEAK | 2 | 1 | y8 | 559.3 | 903.5 | 19.7 |
| | | | 2 | 1 | y7 | 559.3 | 790.4 | 19.7 |
| | | | 2 | 1 | b2 | 559.3 | 215.1 | 19.7 |
| 38 | COL4A2 | GLPGEVLGAQPGPR | 2 | 1 | y6 | 674.4 | 625.3 | 23.9 |
| | | | 2 | 1 | y5 | 674.4 | 554.3 | 23.9 |
| | | | 2 | 1 | y4 | 674.4 | 426.2 | 23.9 |
| 39 | CORO1C | CDLISIPK | 2 | 1 | y4 | 473.3 | 444.3 | 16.7 |
| | | | 2 | 1 | y2 | 473.3 | 244.2 | 16.7 |
| | | | 2 | 1 | b2 | 473.3 | 276.1 | 16.7 |
| 40 | CPN2 | LSNNALSGLPQGVFGK | 2 | 1 | y10 | 801.4 | 989.5 | 28.4 |
| | | | 2 | 1 | y7 | 801.4 | 732.4 | 28.4 |
| | | | 2 | 1 | y3 | 801.4 | 351.2 | 28.4 |
| 41 | CTSD | VSTLPAITLK | 2 | 1 | y7 | 521.8 | 755.5 | 18.4 |
| | | | 2 | 1 | b2 | 521.8 | 187.1 | 18.4 |
| | | | 2 | 1 | b3 | 521.8 | 288.2 | 18.4 |
| 42 | ECM1 | ELLALIQLER | 2 | 1 | y7 | 599.4 | 842.5 | 21.2 |
| | | | 2 | 1 | y6 | 599.4 | 771.5 | 21.2 |
| | | | 2 | 1 | y5 | 599.4 | 658.4 | 21.2 |
| 43 | FCGBP | FAVLQENVAWGNGR | 2 | 1 | y6 | 780.9 | 660.3 | 27.7 |
| | | | 2 | 1 | y5 | 780.9 | 589.3 | 27.7 |
| | | | 2 | 1 | y4 | 780.9 | 403.2 | 27.7 |
| 44 | FSTL1 | LSFQEFLK | 2 | 1 | y7 | 506.3 | 898.5 | 17.8 |
| | | | 2 | 1 | y6 | 506.3 | 811.4 | 17.8 |
| | | | 2 | 1 | b2 | 506.3 | 201.1 | 17.8 |
| 45 | GSTP1 | ASCLYGQLPK | 2 | 1 | y5 | 568.8 | 542.3 | 20.1 |
| | | | 2 | 1 | y2 | 568.8 | 244.2 | 20.1 |
| | | | 2 | 1 | b3 | 568.8 | 319.1 | 20.1 |
| 46 | HRG | DGYLFQLLR | 2 | 1 | y6 | 562.8 | 789.5 | 19.9 |
| | | | 2 | 1 | y5 | 562.8 | 676.4 | 19.9 |
| | | | 2 | 1 | b3 | 562.8 | 336.1 | 19.9 |
| 47 | HSPG2 | SPAYTLVWTR | 2 | 1 | y6 | 597.3 | 775.4 | 21.1 |
| | | | 2 | 1 | y5 | 597.3 | 674.4 | 21.1 |
| | | | 2 | 1 | y3 | 597.3 | 462.2 | 21.1 |
| 48 | ICAM1 | VTLNGVPAQPLGPR | 2 | 1 | y8 | 709.9 | 835.5 | 25.1 |
| | | | 2 | 1 | y5 | 709.9 | 539.3 | 25.1 |
| | | | 2 | 1 | b6 | 709.9 | 584.3 | 25.1 |
| 49 | IGFBP3 | ALAQCAPPPAVCAELVR | 3 | 1 | y6 | 608.3 | 747.4 | 15.5 |
| | | | 3 | 1 | y5 | 608.3 | 587.4 | 15.5 |
| | | | 3 | 1 | b6 | 608.3 | 615.3 | 15.5 |
| 50 | IGFBP3 | YGQPLPGYTTK | 2 | 1 | y8 | 612.8 | 876.5 | 21.7 |
| | | | 2 | 1 | y3 | 612.8 | 349.2 | 21.7 |
| | | | 2 | 1 | b3 | 612.8 | 349.2 | 21.7 |
| 51 | ITIH2 | IQPSGGTNINEALLR | 3 | 1 | y6 | 528.3 | 715.4 | 12.3 |
| | | | 3 | 1 | y4 | 528.3 | 472.3 | 12.3 |
| | | | 3 | 1 | b2 | 528.3 | 242.1 | 12.3 |
| 52 | ITIH4 | LALDNGGLAR | 2 | 1 | y8 | 500.3 | 815.4 | 17.6 |
| | | | 2 | 1 | y6 | 500.3 | 587.3 | 17.6 |
| | | | 2 | 1 | b2 | 500.3 | 185.1 | 17.6 |
| 53 | LDHB | IVVVTAGVR | 2 | 1 | y7 | 457.3 | 701.4 | 16.1 |
| | | | 2 | 1 | y6 | 457.3 | 602.4 | 16.1 |
| | | | 2 | 1 | b2 | 457.3 | 213.2 | 16.1 |

[Table 10-4]

| N. | Gene Symbol | Peptide Sequence | Precursor Charge | Product Charge | Fragment Ion | Precursor (m/z) | Product (m/z) | Collision Energy(V) |
|---|---|---|---|---|---|---|---|---|
| 54 | LDHB | GLTSVINQK | 2 | 1 | y7 | 480.3 | 789.4 | 16.9 |
| | | | 2 | 1 | y4 | 480.3 | 502.3 | 16.9 |
| | | | 2 | 1 | b2 | 480.3 | 171.1 | 16.9 |
| 55 | LRG1 | VAAGAFQGLR | 2 | 1 | y8 | 495.3 | 819.4 | 17.4 |
| | | | 2 | 1 | y7 | 495.3 | 748.4 | 17.4 |
| | | | 2 | 1 | b2 | 495.3 | 171.1 | 17.4 |
| 56 | MBL2 | FQASVATPR | 2 | 1 | y7 | 488.8 | 701.4 | 17.2 |
| | | | 2 | 1 | y6 | 488.8 | 630.4 | 17.2 |
| | | | 2 | 1 | b2 | 488.8 | 276.1 | 17.2 |
| 57 | P4HB | ALAPEYAK | 2 | 1 | y6 | 431.7 | 678.3 | 15.2 |
| | | | 2 | 1 | y5 | 431.7 | 607.3 | 15.2 |
| | | | 2 | 1 | y3 | 431.7 | 381.2 | 15.2 |
| 58 | PDCD4 | GDSVSDSGSDALR | 2 | 1 | y9 | 633.3 | 907.4 | 22.4 |
| | | | 2 | 1 | y7 | 633.3 | 705.4 | 22.4 |
| | | | 2 | 1 | y6 | 633.3 | 618.3 | 22.4 |
| 59 | PKM2 | CDENILWLDYK | 2 | 1 | y5 | 734.8 | 724.4 | 26 |
| | | | 2 | 1 | y4 | 734.8 | 538.3 | 26 |
| | | | 2 | 1 | y2 | 734.8 | 310.2 | 26 |
| 60 | PPBP | ICLDPDAPR | 2 | 1 | y7 | 528.8 | 783.4 | 18.6 |
| | | | 2 | 1 | y6 | 528.8 | 670.3 | 18.6 |
| | | | 2 | 1 | b2 | 528.8 | 274.1 | 18.6 |
| 61 | PTPRJ | DTEVLLVGLEPGTR | 2 | 1 | y7 | 749.9 | 729.4 | 26.6 |
| | | | 2 | 1 | y4 | 749.9 | 430.2 | 26.6 |
| | | | 2 | 1 | b6 | 749.9 | 671.4 | 26.6 |
| 62 | SERPINC1 | VAEGTQVLELPFK | 2 | 1 | y6 | 715.9 | 746.4 | 25.3 |
| | | | 2 | 1 | y4 | 715.9 | 504.3 | 25.3 |
| | | | 2 | 1 | y3 | 715.9 | 391.2 | 25.3 |
| 63 | SERPINC1 | IEDGFSLK | 2 | 1 | y7 | 454.7 | 795.4 | 16 |
| | | | 2 | 1 | y6 | 454.7 | 666.3 | 16 |
| | | | 2 | 1 | b2 | 454.7 | 243.1 | 16 |
| 64 | SFTPB | FLEQECNVLPLK | 2 | 1 | y7 | 745.4 | 843.5 | 26.4 |
| | | | 2 | 1 | y4 | 745.4 | 470.3 | 26.4 |
| | | | 2 | 1 | y3 | 745.4 | 357.2 | 26.4 |
| 65 | SOD3 | VTGVVLFR | 2 | 1 | y6 | 445.8 | 690.4 | 15.7 |
| | | | 2 | 1 | y4 | 445.8 | 534.3 | 15.7 |
| | | | 2 | 1 | b2 | 445.8 | 201.1 | 15.7 |
| 66 | SPARC | NVLVTLYER | 2 | 1 | y7 | 553.8 | 893.5 | 19.5 |
| | | | 2 | 1 | b2 | 553.8 | 214.1 | 19.5 |
| | | | 2 | 1 | b3 | 553.8 | 327.2 | 19.5 |
| 67 | THBS1 | GFLLLASLR | 2 | 1 | y5 | 495.3 | 559.4 | 17.4 |
| | | | 2 | 1 | y4 | 495.3 | 446.3 | 17.4 |
| | | | 2 | 1 | b4 | 495.3 | 431.3 | 17.4 |
| 68 | TXN | CMPTFQFFK | 2 | 1 | y7 | 603.3 | 914.5 | 21.3 |
| | | | 2 | 1 | y5 | 603.3 | 716.4 | 21.3 |

The scope of protection is defined by the appended claims.

All the technical terms used in the present invention are used in the same sense as those generally understood by one skilled in the art related to the present invention, unless otherwise defined.

**Claims**

1. A method for diagnosing pancreatic cancer, comprising:

    measuring the expression level of a combination of biomarkers from blood isolated from a subject; and correlating with pancreatic cancer by comparing the measurement result with a measurement result corresponding to the corresponding marker of a control sample,
    wherein the combination of biomarkers is TTHY, C4BPB, CLU, C1R, ECM1, SERPINA5 (IPSP), LDHB, COL4A2, SERPINC1, VIM, ICAM1, IGFBP2, C4BPA, C5, PPBP, C1S, LRG1, APOH, C7 and IGFBP3, and
    wherein the correlating with pancreatic cancer further comprises determining the subject to have pancreatic cancer when the expression level increases or decreases in comparison with the expression level measured in the control, as a result of measuring the subject,
    the increasing marker is C1R, C1S, C4BPA, C4BPB, C5, C7, ICAM1, IGFBP2, LDHB, LRG1, PPBP, SERPINC1 and VIM, and
    the decreasing marker is APOH, CLU, COL4A2, ECM1, IGFBP3, IPSP, and TTHY; and
    wherein the measuring the expression level is detecting the expression level of the biomarker at a protein or nucleic acid level,
    wherein the protein level detection is carried out by western blot, ELISA, radioimmunoassay, immunodiffusion, immunoelectrophoresis, tissue immunostaining, immunoprecipitation assay, complement fixation assay, FACS, mass spectrometry, or protein array; and
    wherein the nucleic acid detection is carried out by polymerase chain reaction, reverse transcription-polymerase chain reaction, competitive polymerase chain reaction, nuclease protection analysis (RNase, S1 nuclease assay), in situ cross method, nucleic acid microarray or northern blot.

2. The method of claim 1, wherein the combination of biomarkers further comprises CA19-9.

3. The method of claim 1 or 2, wherein the mass spectrometry comprises tandem mass spectrometry, ion trap mass spectrometry, triple quadrupole mass spectrometry, hybrid ion trap/quadrupole mass spectrometry or time-of-flight mass spectrometry.

4. The method of claim 3, wherein a mode used for the mass spectrometry is MRM, and a peptide used in the MRM analysis is as shown in the following table:

| Gene Symbol | Peptide Sequence | Gene Symbol | Peptide Sequence |
|---|---|---|---|
| TTHY | AADDTWEPFASGK | ICAM1 | LLGIETPLPK |
| | | | VTLNGVPAQPLGPR |
| C4BPB | ALLAFQESK | IGFBP2 | LIQGAPTIR |
| | | | ALAQCAPPPAVCAELVR |
| CLU | TLLSNLEEAK | C4BPA | TWYPEVPK |
| | ASSIIDELFQDR | | LSLEIEQLELOR |
| C1R | DYFIATCK | C5 | NADYSYSVWK |
| | | | GGSASTWLTAFALR |
| ECM1 | ELLALIQLER | PPBP | NIQSLEVIGK |
| | | | ICLDPDAPR |
| SERPINA5 (IPSP) | GFQQLLQELNQPR | C1S | TNFDNDIALVR |
| LDHB | IVVVTAGVR | LRG1 | DLLLPQPDLR |
| | GLTSVINQK | | VAAGAFQGLR |
| COL4A2 | GLPGEVLGAQPGPR | APOH | VCPFAGILENGAVR |
| SERPINC1 | VAEGTQVLELPFK | C7 | VLFYVDSEK |
| | IEDGFSLK | | |

(continued)

| Gene Symbol | Peptide Sequence | Gene Symbol | Peptide Sequence |
|---|---|---|---|
| VIM | ILLAELEQLK | IGFBP3 | YGQPLPGYTTK |

**Patentansprüche**

1. Verfahren zur Diagnose von Bauchspeicheldrüsenkrebs, das umfasst:

   Messen des Expressionsniveaus einer Kombination von Biomarkern aus Blut, das von einem Subjekt isoliert wurde, und
   Korrelieren mit Bauchspeicheldrüsenkrebs durch Vergleichen des Messergebnisses mit einem Messergebnis, das dem entsprechenden Marker einer Kontrollprobe entspricht,
   wobei die Kombination von Biomarkern TTHY, C4BPB, CLU, C1R, ECM1, SERPINA5 (IPSP), LDHB, COL4A2, SERPINC1, VIM, ICAM1, IGFBP2, C4BPA, C5, PPBP, C15, LRG1, APOH, C7 und IGFBP3 ist, und
   wobei das Korrelieren mit Bauchspeicheldrüsenkrebs ferner das Bestimmen umfasst, dass das Subjekt Bauchspeicheldrüsenkrebs aufweist, wenn das Expressionsniveau infolge der Messung beim Subjekt im Vergleich zu dem in der Kontrollprobe gemessenen Expressionsniveau erhöht oder erniedrigt ist,
   wobei die erhöhten Marker C1R, C1S, C4BPA, C4BPB, C5, C7, ICAM1, IGFBP2, LDHB, LRG1, PPBP, SERPINC1 und VIM sind, und
   wobei die erniedrigten Marker APOH, CLU, COL4A2, ECM1, IGFBP3, IPSP und TTHY sind, und
   wobei das Messen des Expressionsniveaus das Detektieren des Expressionsniveaus des Biomarkers auf Protein- oder Nukleinsäureebene ist,
   wobei die Detektion auf Proteinebene mittels Western-Blot, ELISA, Radioimmunoassay, Immundiffusion, Immunelektrophorese, Gewebe-Immunfärbung, Immunpräzipitationsassay, Komplementfixationsassay, FACS, Massenspektrometrie oder Protein-Array durchgeführt wird, und
   wobei die Detektion auf Nukleinsäureebene mittels Polymerase-Kettenreaktion, Reverse-Transkription-Polymerase-Kettenreaktion, kompetitiver Polymerase-Kettenreaktion,
   Nuklease-Schutzanalyse (RNase-, S1-Nuklease-Assay), In-situ-Hybridisierung, Nukleinsäure-Mikroarray oder Northern-Blot durchgeführt wird.

2. Verfahren nach Anspruch 1, wobei die Kombination von Biomarkern ferner CA19-9 umfasst.

3. Verfahren nach Anspruch 1 oder 2, wobei die Massenspektrometrie Tandem-Massenspektrometrie, Ionenfallen-Massenspektrometrie, Triple-Quadrupol-Massenspektrometrie, hybride Ionenfallen/Quadrupol-Massenspektrometrie oder Flugzeit-Massenspektrometrie umfasst.

4. Verfahren nach Anspruch 3, wobei ein für die Massenspektrometrie verwendeter Modus MRM ist und ein in der MRM-Analyse verwendetes Peptid in der folgenden Tabelle dargestellt ist:

| Gen-symbol | Peptidsequenz | Gen-symbol | Peptidsequenz |
|---|---|---|---|
| TTHY | AADDTWEPFASGK | ICAM1 | LLGIETPLPK |
| | | | VTLNGVPAQPLGPR |
| C4BPB | ALLAFQESK | IGFBP2 | LIQGAPTIR |
| | | | ALAQCAPPPAVCAELVR |
| CLU | TLLSNLEEAK | C4BPA | TWYPEVPK |
| | ASSIIDELFQDR | | LSLEIEQLELQR |
| C1R | DYFIATCK | C5 | NADYSYSVWK |
| | | | GGSASTWLTAFALR |
| ECM1 | ELLALIQLER | PPBP | NIQSLEVIGK |
| | | | ICLDPDAPR |

(continued)

| Gen-symbol | Peptidsequenz | Gen-symbol | Peptidsequenz |
|---|---|---|---|
| SERPINA5 (IPSP) | GFQQLLQELNQPR | C1S | TNFDNDIALVR |
| LDHB | IVVVTAGVR | LRG1 | DLLLPQPDLR |
| | GLTSVINQK | | VAAGAFQGLR |
| COL4A2 | GLPGEVLGAQPGPR | APOH | VCPFAGILENGAVR |
| SERPINC1 | VAEGTQVLELPFK | C7 | VLFYVDSEK |
| | IEDGFSLK | | |
| VIM | ILLAELEQLK | IGFBP3 | YGQPLPGYTTK |

**Revendications**

1. Méthode permettant le diagnostic du cancer du pancréas, comprenant :

la mesure du niveau d'expression d'une combinaison de biomarqueurs à partir de sang prélevé chez un sujet ; et la mise en corrélation avec le cancer du pancréas par la comparaison du résultat de mesure avec un résultat de mesure correspondant au marqueur correspondant d'un échantillon témoin,
ladite combinaison de biomarqueurs étant TTHY, C4BPB, CLU, C1R, ECM1, SERPINA5 (IPSP), LDHB, COL4A2, SERPINC1, VIM, ICAM1, IGFBP2, C4BPA, C5, PPBP, C1S, LRG1, APOH, C7 et IGFBP3 ; et
ladite mise en corrélation avec le cancer du pancréas comprenant en outre la détermination que le sujet souffre du cancer du pancréas lorsque le niveau d'expression augmente ou diminue par rapport au niveau d'expression mesuré dans le témoin, à la suite de la mesure du sujet,
ledit marqueur croissant étant C1R, C1S, C4BPA, C4BPB, C5, C7, ICAM1, IGFBP2, LDHB, LRG1, PPBP, SERPINC1 et VIM, et
ledit marqueur décroissant étant APOH, CLU, COL4A2, ECM1, IGFBP3, IPSP et TTHY ; et
ladite mesure du niveau d'expression consistant à détecter le niveau d'expression du biomarqueur à l'échelle des protéines ou des acides nucléiques,
ladite détection à l'échelle des protéines étant effectuée par transfert Western, dosage ELISA, dosage radio-immunologique, immunodiffusion, immunoélectrophorèse, immunocoloration d'un tissu, dosage d'immunoprécipitation, test de fixation du complément, FACS, spectrométrie de masse ou puce à protéine ; et
ladite détection des acides nucléiques étant réalisée par réaction en chaîne par polymérase, réaction en chaîne par transcription inverse-polymérase, réaction en chaîne par polymérase compétitive, analyse de protection par une nucléase (RNase, test à la nucléase S1), méthode croisée in situ, micropuce à acide nucléique ou transfert Northem.

2. Méthode selon la revendication 1, ladite combinaison de biomarqueurs comprenant en outre CA19-9.

3. Méthode selon la revendication 1 ou 2, ladite spectrométrie de masse comprenant la spectrométrie de masse en tandem, la spectrométrie de masse à piège à ions, la spectrométrie de masse à triple quadripôle, la spectrométrie de masse hybride à piège à ions/quadripôle ou la spectrométrie de masse à temps de vol.

4. Méthode selon la revendication 3, un mode utilisé pour la spectrométrie de masse étant le mode MRM, et le peptide utilisé dans l'analyse par MRM étant comme indiqué dans le tableau suivant :

| Symbole du gène | Séquence peptidique | Symbole du gène | Séquence peptidique |
|---|---|---|---|
| TTHY | AADDTWEPFASGK | ICAM1 | LLGIETPLPK |
| | | | VTLNGVPAQPLGPR |
| C4BPB | ALLAFQESK | IGFBP2 | LIQGAPTIR |
| | | | ALAQCAPPPAVCAELVR |

(continued)

| Symbole du gène | Séquence peptidique | Symbole du gène | Séquence peptidique |
|---|---|---|---|
| CLU | TLLSNLEEAK | C4BPA | TWYPEVPK |
| CLU | ASSIIDELFQDR | C4BPA | LSLEI EQLELOR |
| C1R | DYFIATCK | C5 | NADYSYSVWK |
| C1R | DYFIATCK | C5 | GGSASTWLTAFALR |
| ECM1 | ELLALIQLER | PPBP | NIQSLEVIGK |
| ECM1 | ELLALIQLER | PPBP | ICLDPDAPR |
| SERPINA5 (IPSP) | GFQQLLQELNQPR | C1S | TNFDNDIALVR |
| LDHB | IVWWTAGVR | LRG1 | DLLLPQPDLR |
| LDHB | GLTSVINQK | LRG1 | VAAGAFQGLR |
| COL4A2 | GLPGEVLGAQPGPR | APOH | VCPFAGILENGAVR |
| SERPINC1 | VAEGTQVLELPFK | C7 | VLFYVDSEK |
| SERPINC1 | IEDGFSLK | C7 | VLFYVDSEK |
| VIM | ILLAELEQLK | IGFBP3 | YGQPLPGYTTK |

【Figure】
【FIG. 1】

【FIG. 2】

$$R(z) = max(0,z)$$

【FIG. 3】

Structure of triple Quadruple Mass spectrometry

【FIG. 4】

【FIG. 5】

【FIG. 6】

【FIG. 7】

【FIG. 8】

【FIG. 9】

A.

B.

C.

D.

【FIG. 10】

## Distribution of Skewness

【FIG. 11】

【FIG. 12】

【FIG. 13】

【FIG. 14】

【FIG. 15】

【FIG. 16】

EP 3 998 480 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20150030046
- US 5599695 A
- US 5556752 A
- US 5631734 A

**Non-patent literature cited in the description**

- Identification and verification of transthyretin as a potential biomarker for pancreatic ductal adenocarcinoma. **CHEN JIONG et al.** JOURNAL OF CANCER RESEARCH AND CLINICAL ONCOLOGY. SPRINGER INTERNATIONAL, 01 July 2013, vol. 139, 1117-1127
- **COLYER et al.** *J Chromatogr A.*, 1997, vol. 781 (1-2), 271-6
- **PETRICOIN et al.** *Lancet*, 2002, vol. 359, 572-77
- **CHAN-HUI et al.** *Clinical Immunology*, 2004, vol. 111, 162-174
- **MONTAGNE et al.** *Eur J Clin Chem Clin Biochem.*, 1992, vol. 30, 217-22
- **KIM et al.** *J Proteome Res.*, 2010, vol. 9, 689-99
- **ANDERSON, L et al.** *Mol Cell Proteomics*, 2006, vol. 5, 573-88
- Enzyme Immunoassay. CRC Press, 1980
- **GAASTRA, W.** Enzyme-linked immunosorbent assay(ELISA), in Methods in Molecular Biology. Humana Press, 1984, vol. 1
- **HAN, H. et al.** *Cancer Res.*, 2002, vol. 62, 2890-6
- **SCHENA et al.** *Proc Natl Acad Sci USA.*, 1996, vol. 93 (20), 10614-9
- **SCHENA et al.** *Science*, 1995, vol. 270 (5235), 467-70